# EUROPEAN PATENT APPLICATION

(11) **EP 3 714 942 A1**
(43) Date of publication of application: **30.09.2020**
(21) Application number: 19305447.5
(22) Date of filing: 05.04.2019
(51) Int. Cl.: A61P 35/00, A61P 37/00, C07K 16/28, A61K 39/395

(54) **MONOCLONAL ANTIBODY AGAINST STIM1**

(30) Priority: 25.03.2019 EP 19165024
(71) Applicant: Université De Bretagne Occidentale - UBO, 29200 Brest (FR); Centre Hospitalier Régional et Universitaire de Brest, 29609 Brest Cedex (FR); Inserm, 75013 Paris (FR)
(72) Inventor: MIGNEN, Olivier, 29460 Logonna Daoulas (FR); RENAUDINEAU, Yves, 29200 Brest (FR); DEBANT, Marjolaine, 64700 Hendaye (FR); HEMON, Patrice, 29800 Saint Divy (FR)
(74) Representative: Cabinet Becker et Associés

(57) **Abstract**

The present invention relates to a compound that specifically binds to the region between amino acid residues 58 and 201, preferably 97 and 127 of the human STIM1 amino acid sequence SEQ ID NO: 1.

The present invention also relates to a composition comprising a therapeutically effective amount of the compound, a host cell that produces an isolated antibody, an isolated nucleic acid sequence encoding the isolated antibody and an expression vector comprising the nucleic acid.

The present invention additionally relates to a method of producing the isolated antibody, to the isolated antibody for its use as a drug, especially for its use in treating a condition or a disorder in which the STIM1 protein localized to the plasma membrane of the cells is overexpressed, and to an isolated protein fragment consisting of the region between amino acid residues 97 and 127 for developing modulators of the STIM1 amino acid sequence SEQ ID NO: 1.

SEQUENCE LISTING

<110>  Université de Bretagne Occidentale - UBO
       Centre Hospitalier Régional et Universitaire de Brest
       INSERM

<120>  MONOCLONAL ANTIBODY AGAINST STIM1

<130>  BNT225732EP01

<150>  EP19165024.1
<151>  2019-03-25

<160>  19

<170>  PatentIn version 3.5

<210>  1
<211>  685
<212>  PRT
<213>  Homo sapiens

<400>  1


<210>  2
<211>  31
<212>  PRT
<213>  Homo sapiens

<400>  2

Asn Tyr His Asp Pro Thr Val Lys His Ser Thr Phe His Gly Glu Asp
1               5                   10                  15


Lys Leu Ile Ser Val Glu Asp Leu Trp Lys Ala Trp Lys Ser Ser
            20                  25                  30


<210>  3
<211>  5
<212>  PRT
<213>  Mus musculus

<400>  3

Arg Tyr Ser Met Ser
1               5


<210>  4
<211>  17
<212>  PRT
<213>  Mus musculus

<400>  4

His Ile Arg Asn Gly Gly Gly Ser Thr Tyr Tyr Pro Asp Thr Val Lys
1               5                   10                  15


Gly


<210>  5
<211>  12
<212>  PRT
<213>  Mus musculus

<400>  5

Pro Pro Tyr Gly Asn Tyr Glu Gly Tyr Phe Asp Val
1               5                   10


<210>  6
<211>  17
<212>  PRT
<213>  Mus musculus

<400>  6

Lys Ser Ser Gln Ser Leu Leu Asn Ser Arg Thr Arg Lys Asn Tyr Leu
1               5                   10                  15

Ala

<210>  7
<211>  7
<212>  PRT
<213>  Mus musculus

<400>  7

Trp Ala Ser Thr Arg Glu Ser
1               5

<210>  8
<211>  8
<212>  PRT
<213>  Mus musculus

<400>  8

Lys Gln Ser Tyr Asn Leu Tyr Thr
1               5

<210>  9
<211>  112
<212>  PRT
<213>  Unknown

<220>
<223>  artificial sequence

<400>  9

Asp Ile Val Met Ser Gln Ser Pro Ser Ser Leu Ala Val Ser Ala Gly
1               5                   10                  15

Glu Lys Val Thr Met Ser Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Lys Gln
                85                  90                  95

Ser Tyr Asn Leu Tyr Thr Phe Gly Gly Gly Thr Lys Leu Glu Ile Lys
            100                 105                 110

<210>  10
<211>  121
<212>  PRT
<213>  Unknown

<220>
<223>  artificial sequence

<400>  10

Glu Val Lys Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Arg Tyr
            20                  25                  30

Ser Met Ser Trp Val Arg Gln Thr Pro Glu Lys Arg Leu Glu Trp Val
        35                  40                  45

Ala His Ile Arg Asn Gly Gly Gly Ser Thr Tyr Tyr Pro Asp Thr Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg Pro Pro Tyr Gly Asn Tyr Glu Gly Tyr Phe Asp Val Trp Gly
            100                 105                 110

Ala Gly Thr Thr Val Thr Val Ser Ser
        115                 120

<210>  11
<211>  15
<212>  DNA
<213>  Mus musculus

<400>  11
aggtattcca tgtct                                                        15

<210>  12
<211>  51
<212>  DNA
<213>  Mus musculus

<400> 12
cacattagaa atggtggtgg tagcacctac tatccagaca ctgtaaaggg c 51

<210> 13
<211> 36
<212> DNA
<213> Mus musculus

<400> 13
   cccccctatg gtaactacga ggggtacttc gatgtc 36

<210> 14
<211> 51
<212> DNA
<213> Mus musculus

<400> 14
aaatccagtc agagtctgct caacagtaga acccgaaaga actacttggc t 51

<210> 15
<211> 21
<212> DNA
<213> Mus musculus

<400> 15
tgggcatcca ctagggaatc t 21

<210> 16
<211> 24
<212> DNA
<213> Mus musculus

<400> 16
aagcaatctt ataatctgta cacg 24

<210> 17
<211> 336
<212> DNA
<213> Unknown

<220>
<223> artificial sequence

<400> 17
gacattgtga tgtcacagtc tccatcctcc ctggctgtgt cagcaggaga gaaggtcact 60

atgagctgca aatccagtca gagtctgctc aacagtagaa cccgaaagaa ctacttggct 120

tggtaccagc agaaaccagg gcagtctcct aaactgctga tctactgggc atccactagg 180

gaatctgggg tccctgatcg cttcacaggc agtggatctg ggacagattt cactctcacc 240

atcagcagtg tgcaggctga agacctggca gtttattact gcaagcaatc ttataatctg 300

tacacgttcg gaggggggac caagctggaa ataaaa 336

<210>  18
<211>  363
<212>  DNA
<213>  Unknown

<220>
<223>  artificial sequence

<400>  18
   gaagtgaagc tggtggagtc tgggggaggt ttagtgcagc ctggagggtc cctgaaactc      60
   tcctgtgcag cctctggatt cactttcagt aggtattcca tgtcttgggt tcgccagact     120
   ccagagaaga ggctggagtg ggtcgcacac attagaaatg gtggtggtag cacctactat     180
   ccagacactg taaagggccg attcaccatc tccagagaca atgccaagaa caccctgtac     240
   ctgcaaatga gcagtctgaa gtctgaggac acggccatgt attactgtgc aagacccccc     300
   tatggtaact acgaggggta cttcgatgtc tggggcgcag ggaccacggt caccgtctcc     360
   tca                                                                   363


<210>  19
<211>  34
<212>  PRT
<213>  Homo sapiens

<400>  19

Lys Leu Ser Phe Glu Ala Val Arg Asn Ile His Lys Leu Met Asp Asp
1               5                   10                  15


Asp Ala Asn Gly Asp Val Asp Val Glu Glu Ser Asp Glu Phe Leu Arg
            20                  25                  30


Glu Asp

## Description

### Technical field

The present invention refers to a compound that specifically binds to a particular region of the STIM1 amino acid sequence SEQ ID NO: 1.

Therefore, the present invention has utility in pharmaceutical field.

In the description below, the references into brackets ([ ]) refer to the listing of references situated at the end of the text.

### Background of the Invention

Taken separately, each autoimmune disease is a rare disease with a case in the tens of thousands. But together, these autoimmune diseases represent the third leading cause of morbidity in industrialized countries after cancer and cardiovascular disease. Emblematic examples of this singularity are systemic lupus erythematosus (SLE), which suffers from being very complicated to detect, since each patient presents a particular profile, and chronic lymphocytic leukaemia (CLL). Both Systemic lupus erythematosus (SLE) and chronic lymphocytic leukaemia (CLL) are still incurable.

SLE is a heterogeneous disease, of autoimmune origin, characterized by the presence of autoreactive lymphocytes and of antinuclear auto-antibodies (ANA). It is a multisystemic disease, with very varied clinical manifestations. Prevalence varies in different ethnic groups, but is estimated at about 1 in 10,000, with a male/female ratio of 10:1. The clinical heterogeneity of this disease reflects its aetiopathogenic complexity, comprising both genetic and environmental factors. SLE may affect all organs. The most common manifestations are rash, arthritis and fatigue. The most severe manifestations include nephritis, neurological disorders, anaemia and thrombocytopenia. More than 90% of patients have ANAs that are considered positive above 1/160th. SLE is a disease with episodic evolution. The aims of the current treatment are: treat the acute episodes that may compromise the vital prognosis, minimize the risks of flare-ups during periods of relative stability and monitor the symptoms which, although not jeopardizing the vital prognosis, affect everyday quality of life.

Hydroxychloroquine and non-steroidal anti-inflammatory drugs are indicated in the moderate forms of SLE; the corticoids and immunosuppressants are reserved for the most severe forms; the anti-CD20 monoclonal antibody (Rituximab, Mabthera®) that targets the B lymphocytes (B cells), and the anti-BLYS (Belimumab) are currently indicated in patients who are more severely affected and / or have not responded to the usual treatments. Despite the improvement in prognosis after the introduction of corticoids and immunosuppressants, SLE continues to have a significant impact on patient morbidity and mortality.

CLL is a chronic malignant haemopathy that also affects the B cells. These cells play an important role at the immune system level. In the course of CLL, the B cells of CLL are blocked in their life cycle, when they reach maturity, and their production continues. Consequently, these tumoral B cells eventually accumulate in the blood, in the lymph nodes, spleen, liver and bone marrow, which leads to an increase in volume of the secondary lymphatic organs. The treatments currently available against CLL are most often used when the disease is at an advanced stage. In terms therapy, as the leukaemic B cells are CD20+, a monoclonal antibody specifically recognizing this target may be used in the treatment (rituximab, Mabthera®), which is associated with chemotherapeutic products such as fludarabine, cyclophosphamide, bendamustine, chlorambucil, and lenalinomide. Another target is Bruton's tyrosine kinase that is specific for the B cells whose expression is increased in the leukaemic cells. Ibrutinib, an inhibitor of this enzyme, leads to apoptosis of the leukaemic cells, giving longer remissions, even in the refractory or recurring forms. Other effective oral targeted therapies used in CLL are phosphatidyl-inositol 3-kinase delta (PI3K-delta) inhibitors such as idelalisib, and Bcl-2-specific BH3-mimetic like venetoclax. However, the treatments may give exposure to undesirable side effects and for some patients a high risk of relapse is reported.

In the pathology of SLE and CLL, a disturbance of calcium signaling of the B cells in SLE and CLL is described following stimulation of the B-cell antigen receptor (BCR). In addition to these defects of calcium signaling, the B cells of SLE are characterized by a deficiency of production of interleukin 10 (IL-10), which affects the activity of the regulatory B lymphocytes (Bregs). This deficiency of activity of the Bregs in SLE leads to less regulation of T lymphocyte (T cell) proliferation, which might again contribute to amplifying the autoimmunity and tumoral process.

In both of these disorders, the B cell represents the main therapeutic target. However, some patients do not respond to the existing treatments.

Previously, the Applicant has identified the fraction of the plasma membrane-specific STIM1 protein (mSTIM1) as a therapeutic target in LES and CLL (document EP2982982 **([1])**)**.** In document EP3062105 **([2]),** they have described a diagnostic method of SLE and / or CLL, comprising the *in vitro* detection of the expression of the membrane fraction of the STIM1 protein. They also disclose a method for predicting the progression and / or monitoring of the progression of CLL and / or SLE, comprising the *in vitro* detection of the expression of the fraction of the STIM1 protein located at the cellular plasma membrane.

However, a need exists of a therapeutic alternative to the current treatments of SLE and CLL, and advantageously to other autoimmune diseases. The present invention fulfills these and other needs.

### Description of the invention

The Applicant has found surprisingly that a new antibody, specifically targeting the membrane fraction of the STIM1 protein (hereinafter referred to as a "mSTIM1"), constitutes a new therapeutic target for SLE and CLL.

The Applicant has demonstrated that mSTIM1 can be recognized by the antibody of the invention and that the binding of the antibody of the invention to mSTIM1 modulates the cellular responses of the B lymphocyte, thus providing a new therapeutic solution in CLL and SLE. So, the present invention proposes to use the antibody of the invention to modulate mSTIM1 activity.

Furthermore, the results of the Applicant's work lead to the conclusion that the constitutive entry of calcium is involved in the survival of B cells, to the secretion of antibodies by B cells, and migration of B cells, and that these three cellular processes are strongly deregulated in B cells during CLL and/or SLE. Moreover, the Applicant has been able to demonstrate that this pathway is regulated by the mSTIM1 protein. Advantageously, the anti-STIM1 antibody of the invention targets the constitutive entry of calcium in patients suffering from LES and CLL. The modulation of a a calcium entry independent of the B-cell antigen receptor (BCR) pathway is therefore a completely new and innovative therapeutic approach that may offer an alternative to existing therapies, improve their effectiveness and reduce their effects.

Surprisingly, the results of the Applicant's work lead to the conclusion that the amplitude of the calcium entry as well as the amount of mSTIM1 of the B cells are correlated with the evolution of CLL and SLE.

The use of anti-STIM1 antibody of the invention may constitute a new immunotherapy that advantageously modulates an alternative signaling pathway to currently targeted signaling pathways in the treatment of CLL and SLE.

Accordingly, in a first aspect, the present invention provides a compound that specifically binds to the region between amino acid residues 58 and 201 of the STIM1 amino acid sequence SEQ ID NO: 1 and modulates STIM1 activity.

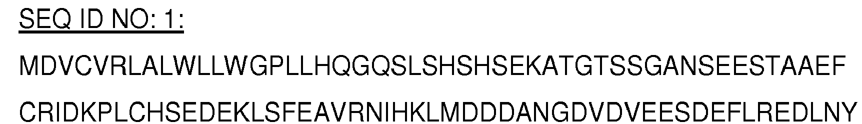

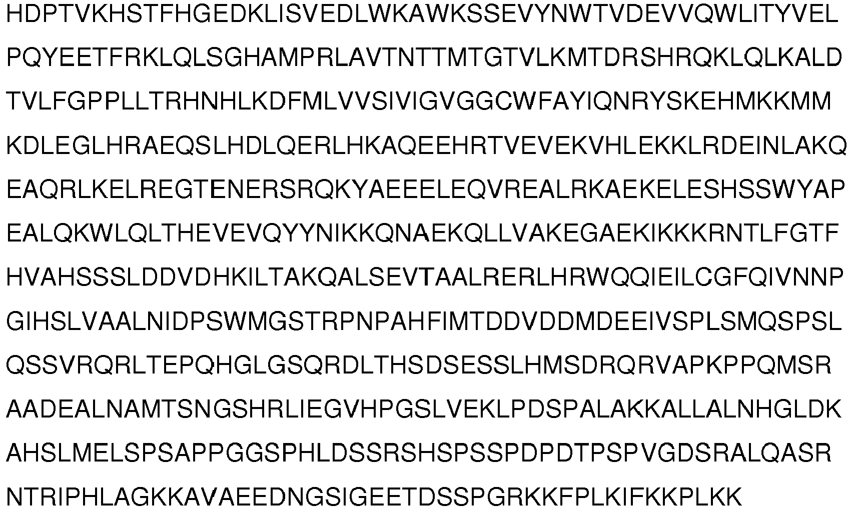

Advantageously, the compound specifically binds to the region between amino acid residues 97 and 127, especially 113 and 122, of the STIM1 amino acid sequence SEQ ID NO: 1 and modulates STIM1 activity.

The term "compound" as used therein refers to any compound that binds to the region between amino acid residues 58 and 201, preferably between amino acid residues 97 and 127 or 113 and 122 of STIM1 and modulates its biological activity. The modulation may be any type of modulation, as long as the binding of the compound of the invention has an effect on STIM1 activity. For example, the compound of the invention may be an activator or an inhibitor of STIM1 activity. The compound of the invention may be of any kind, for example it may be chosen from the group comprising isolated antibodies, isolated antibody fragments, proteins, peptides, chemical compounds, aptamer. Preferably, the compound may be an isolated antibody. The effect of the compound of the invention on STIM1 activity, localization or function may be measured by any technique known by the skilled person, for example calcium flux and signaling measurements, luminescence of fluorescence complementation assays, flow cytometry. Also, the binding of the compound of the invention may be measured by any known techniques, for example by FACS (Fluorescence activated cell sorting) or Biacore™ assay, Octet™ assay, OpenSPR™, any mass spectrometry analysis.

Specific binding between two entities means a binding with an equilibrium constant (K_{A}) (kₒₙ/k_{off}) of at least 10²M⁻¹.

The phrase "specifically (or selectively) binds to" refers to a binding reaction that is determinative of the presence of the antigen, i.e. the region between amino acid residues 58 and 201, preferably between amino acid residues 97 and 127 of the STIM1 amino acid sequence SEQ ID NO: 1, in a heterogeneous population of proteins and other biologics. In addition to the equilibrium constant (K_{A}) noted above, the compound of the invention may advantageously also have a dissociation rate constant (K_{D}) (k_{off}/kₒₙ) of less than 5x10⁻²M and binds to the antigen as defined above with an affinity that is at least twofold greater than its affinity for binding to a nonspecific antigen.

In one embodiment, the compound of the invention has dissociation constant (K_{d}) of less than 3000 pM as assessed using a method described herein or known to one of skill in the art (e.g., a BIAcore™ assay, ELISA, FACS, SET) (Biacore™ International AB, Uppsala, Sweden). The term "K_{assoc}" or "Kₐ", as used herein, refers to the association rate of a particular antibody-antigen interaction, whereas the term "K_{dis}" or "K_{d}," as used herein, refers to the dissociation rate of a particular antibody-antigen interaction. The term "K_{D}", as used herein, refers to the dissociation constant, which is obtained from the ratio of K_{d} to Kₐ (i.e. K_{d}/Kₐ) and is expressed as a molar concentration (M). K_{D} values for antibodies can be determined using methods well established in the art. A method for determining the K_{D} of an antibody is by using surface plasmon resonance, or using a biosensor system such as a Biacore® system.

The compound of the invention may be identified by a method comprising the steps of:
(i) providing a biological sample such as isolated intact cells expressing on their surface the STIM1 protein localized to the plasma membrane, or a isolated peptide containing the region between amino acid residues 58 and 201, preferably between amino acid residues 97 and 127 of STIM1;
(ii) contacting the region between amino acid residues 58 and 201, preferably between amino acid residues 97 and 127 of STIM1 and a compound to be tested under conditions allowing to test the binding and/or the biological effect of the compound upon the region between amino acid residues 58 and 201, preferably between amino acid residues 97 and 127 of STIM1 ; and
(iiia) determining the biological effect of the compound upon STIM1 using functional and binding assays, thereby identifying and selecting a compound that modulates STIM1 activity, localization of function; and/or
(iiib) determining the binding between the compound and the STIM1 region, thereby identifying and selecting a compound that specifically binds to the region between amino acid residues 58 and 201, preferably between amino acid residues 97 and 127 of STIM1.

The term "antibody" as used herein refers to whole antibodies that interact with, e.g., by binding, steric hindrance, stabilizing/destabilizing, spatial distribution, the region between amino acid residues 58 and 201, preferably between amino acid residues 97 and 127 of the STIM1 amino acid sequence SEQ ID NO: 1, or to an "antibody fragment" , which refers to one or more portions of an antibody that retain the ability to specifically interact with the region between amino acid residues 58 and 201, preferably between amino acid residues 97 and 127 of the STIM1 amino acid sequence SEQ ID NO: 1 as mentioned above. Advantageously, they modulate mSTIM1 function to correct the defects of patways regulated by mSTIM1 and cellular functions in which it is involved. The antibody of the invention may be a naturally occurring antibody, which is a glycoprotein comprising at least two heavy (abbreviated herein as H) chains and two light (L) chains inter-connected by disulfide bonds. Each heavy chain is comprised of a heavy chain variable region (abbreviated herein as VH) and a heavy chain constant region (abbreviated herein as CH). The heavy chain constant region is comprised of three domains, CH1, CH2 and CH3. Each light chain is comprised of a light chain variable region (abbreviated herein as VL) and a light chain constant region (abbreviated herein as CL). The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system as effector cells and the first component (C1q) of the classical complement system.

The term "antibody" or "antibody fragment" include for example, monoclonal antibodies, polyclonal antibodies, human antibodies, humanized antibodies, chimeric antibodies, single-chain Fvs (scFv), disulfide-linked Fvs (sdFv), Fab fragments, F(ab') fragments, and anti-idiotypic (anti-Id) antibodies including, e.g., anti-Id antibodies to antibodies of the invention), a monovalent fragment consisting of the VL, VH, CL and CH1 domains; a F(ab)₂ fragment, F(ab₂)', F(ab)₂', scFv, VHH, a Fd fragment consisting of the VH and CHI domains; a Fv fragment consisting of the VL and VH domains of a single arm of an antibody; a dAb fragment, which consists of a VH domain; and an isolated complementarity determining region (CDR). The antibody fragments may be obtained using conventional techniques known to those of skill in the art, and the fragments are screened for utility in the same manner as are intact antibodies.

The framework and/or constant region of the antibody, in the case of an entire antibody, may be from mammals and non mammals such as human, rodent, camel, canine, feline, shark. Preferably, the constant regions of each of the light chains and each of the heavy chains of the antibody according to the invention are human constant regions. In this preferred embodiment of the invention, the immunogenicity of the antibody is reduced in humans, and consequently, the antibodys' effectiveness is improved upon therapeutic administration to humans.

The antibodies can be of any isotype for example IgG, IgE, IgM, IgD, IgA and IgY, of any class, for example IgGI, IgG2, IgG3, IgG4, IgA1 and IgA2, or of any subclass. Preferably, the antibodies of the invention are IgG1.

According to a preferred embodiment of the invention, the constant region of each of the light chains of the antibody according to the invention is of κ type. Any allotype is suitable for the implementation of the invention, e.g. Km(1), Km(1, 2), Km(1, 2, 3) or Km(3).

The phrase "isolated antibody" as used herein refers to an antibody that has been identified and separated and/or recovered from a component of its natural environment. Contaminant components of its natural environment are materials that would interfere with diagnostic or therapeutic uses for the antibody, and may include enzymes, hormones, and other proteinaceous or non-proteinaceous solutes. Isolated antibody includes the antibody in situ within recombinant cells since at least one component of the antibody's natural environment will not be present. Ordinarily, however, isolated antibody will be prepared by at least one purification step.

"STIM1" refers, according to the present invention, to the stromal interaction molecule 1, also referenced in the literature as "GOK". STIM1 is a protein that in humans is encoded by the *STIM1* gene. STIM1 is a multidomain transmembrane protein. STIM1 is mostly localized at the endoplasmic reticulum membrane, and to a lesser extent at the plasma membrane. Regarding STIM1 located at the ER membrane, the STIM1 N-terminal region is located in the ER lumen and contains a SAM domain (sterile α motif domain, a protein-protein interaction module) and an EF-hand motif (calcium-binding motif). In the middle of the protein, there is a transmembrane domain, which is followed by a cytoplasmic C-terminal region, including a coiled coil, an ERM domain (ezrin-radixin-moesin) and a basic/serine/proline region. The STIM1 amino acid sequence, i.e. the amino acid sequence of the entire protein STIM1, is SEQ ID NO: 1. The peptide sequence of region between amino acid residues 97 and 127 of the STIM1 is given in SEQ ID NO: 2 (NYHDPTVKHSTFHGEDKLISVEDLWKAWKSS). It is a region belonging to the second EF-Hand domain of STIM1.

Advantageously, the isolated antibody of the invention may comprise at least one sequence of the group consisting of:
- a variable heavy (V_{H}) chain complementary determining region (CDR) 1 having the amino acid sequence SEQ ID NO: 3 (RYSMS);
- a variable heavy (V_{H}) chain CDR2 having the amino acid sequence SEQ ID NO: 4 (HIRNGGGSTYYPDTVKG); and
- a variable heavy (V_{H}) chain CDR3 having the amino acid sequence SEQ ID NO: 5 (PPYGNYEGYFDV).

In a particular embodiment, the isolated antibody of the invention may comprise :
- a variable heavy (V_{H}) chain complementary determining region (CDR) 1 having the amino acid sequence SEQ ID NO: 3;
- a variable heavy (V_{H}) chain CDR2 having the amino acid sequence SEQ ID NO: 4; and
- a variable heavy (V_{H}) chain CDR3 having the amino acid sequence SEQ ID NO: 5.

Advantageously, the isolated antibody of the invention may comprise at least one sequence of the group consisting of:
- a variable light (V_{L}) chain CDR1 having the amino acid sequence SEQ ID NO: 6 (KSSQSLLNSRTRKNYLA);
- a variable light (V_{L}) chain CDR2 having the amino acid sequence SEQ ID NO: 7 (WASTRES); and
- a variable light (V_{L}) chain CDR3 having the amino acid sequence SEQ ID NO: 8 (KQSYNLYT).

In a particular embodiment, the isolated antibody of the invention may comprise :
- a variable light (V_{L}) chain CDR1 having the amino acid sequence SEQ ID NO: 6;
- a variable light (V_{L}) chain CDR2 having the amino acid sequence SEQ ID NO: 7; and
- a variable light (V_{L}) chain CDR3 having the amino acid sequence SEQ ID NO: 8.

In a particular embodiment, the isolated antibody of the invention comprises:
- a variable heavy (V_{H}) chain CDR 1 having the amino acid sequence SEQ ID NO: 3;
- a variable heavy (V_{H}) chain CDR2 having the amino acid sequence SEQ ID NO: 4;
- a variable heavy (V_{H}) chain CDR3 having the amino acid sequence SEQ ID NO: 5;
- a variable light (V_{L}) chain CDR1 having the amino acid sequence SEQ ID NO: 6;
- a variable light (V_{L}) chain CDR2 having the amino acid sequence SEQ ID NO: 7; and
- a variable light (V_{L}) chain CDR3 having the amino acid sequence SEQ ID NO: 8.

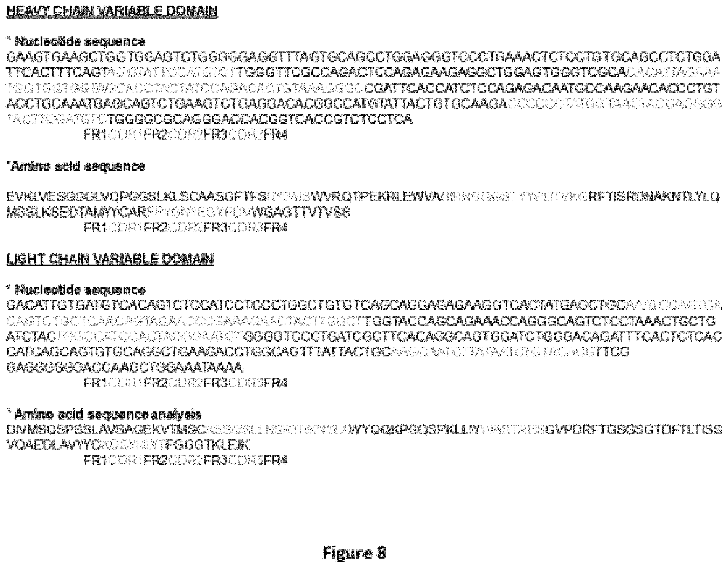

In another particular embodiment, the isolated antibody of the invention may comprise :
a) a variable light chain comprising the sequence SEQ ID NO: 9 (see figure 8); and
b) a variable heavy chain comprising the sequence SEQ ID NO: 10 (see figure 8).

It is understood that the antibody according the invention also extends to conservatively modified variants comprising variation(s) of the above mentioned sequences. Variants may be for example antibodies having one to four amino acid changes in anyone of the above-mentioned sequences.

The phrase "conservatively modified variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the disclosure. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M). In some embodiments, the term "conservative sequence modifications" are used to refer to amino acid modifications that do not significantly affect or alter the binding characteristics of the antibody containing the amino acid sequence.

It is understood that the antibody according the invention also extends to antibodies that cross-compete with an antibody having all or part of the above-mentioned sequences, for example with an antibody having a variable light chain comprising the sequence SEQ ID NO: 9 and a variable heavy chain comprising the sequence SEQ ID NO: 10. The terms "cross-compete" and "cross-competing" are used interchangeably herein to mean the ability of an antibody or other binding agent to interfere with the binding of other antibodies or binding agents to the region between amino acid residues 58 and 201, preferably between amino acid residues 97 and 127 of the STIM1 amino acid sequence SEQ ID NO: 1 in a standard competitive binding assay. The ability or extent to which an antibody or other binding agent is able to interfere with the binding of another antibody or binding molecule to HER3 , and therefore whether it can be said to cross-compete according to the disclosure, can be determined using standard competition binding assays. One suitable assay involves the use of the Biacore™ technology (e.g. by using the BIAcore™ 3000 instrument (Biacore™, Uppsala, Sweden)), which can measure the extent of interactions using surface plasmon resonance technology. Another assay for measuring cross-competing uses an ELISA-based approach.

The antibody according to the invention can be constructed using standard techniques of recombinant DNA, are well known to the person skilled in the art, and more particularly using the techniques of constructing "chimeric" antibodies described for example in Morrison et al., Proc. Natl. Acad. Sci. U.S.A., 81, pp. 6851-55 (1984) **([3])** wherein the recombinant DNA technology is used to replace the CDR region of a heavy chain and/or the constant region of a light chain of an antibody from a non-human mammal with the corresponding regions of a human immunoglobulin. One particular embodiment will be illustrated in more detail.

According to the invention, in the case the antibody is a full antibody, the isolated antibody may be a monoclonal antibody. Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Harlow et al.: "Antibodies: A Laboratory Manual", Cold Spring HarborLaboratory Press, 2nd ed. 1988 **([4]);** Hammerling, et al.: "Monoclonal Antibodies and T-Cell Hybridomas", Elsevier, N. Y., 1981, pp. 563-681 **([5]).**

According to the invention, the isolated antibody if the invention may be a chimeric, a human or a humanized antibody. "Humanized" forms of non-human, e.g. rodent, antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit, or non-human primate having the desired specificity, affinity, and capacity. In some instances, framework region (FR) of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also may comprise at least a portion of an immunoglobulin constant region (Fc), typically that of a human immunoglobulin. For further details, see Jones et al, Nature, 321: 522-525 (1986) **([6]);** Riechmann et al, Nature, 332: 323-329 (1988) **([7]);** and Presta, Curr. Op. Struct. Biol. 2: 593-596 (1992) **([8]).**

Advantageously, the antibody of the invention may be modified in order to be coupled with a drug, for example for enhancing ADCC, or with another antibody, or with a fluorophore (for biomarker), or with any other molecules or ligand such as nanoparticles, metals or radioelements for imaging, and/or for therapeutic and/or for theranostic use.

Another object of the invention is a composition, preferably a pharmaceutical composition, comprising a therapeutically effective amount of a compound of the invention.

The said compositions may be prepared according to techniques commonly available to those skilled in the art. They can be prepared for example by mixing the compound of the invention having the desired degree of purity with optional physiologically acceptable pharmaceutically acceptable carrier, excipients or stabilizers in the form of lyophilised formulations or aqueous solutions. Such pharmaceutical compositions are destined for treating a patient in need.

In order to treat a patient in need, a therapeutically effective dose of the compound may be administered. By "therapeutically effective dose" herein is meant a dose that produces the effects for which it is administered. The exact dose will depend on the purpose of the treatment, and will be ascertainable by one skilled in the art using known techniques. Dosages may range from 0.001 to 100 mg/kg of body weight or greater, for example 0.1, 1.0, 10, or 50 mg/kg of body weight, with 0.1 to 10 mg/kg being preferred..

Administration of the composition of the invention may be done in a variety of ways, including, but not limited to, orally, subcutaneously, intravenously, parenterally, intranasally, intraortically, intraocularly, rectally, vaginally, transdermal, topically (e.g., gels, salves, lotions, creams, etc.), intraperitoneal, intramuscularly, intrapulmonary
The antibody of the invention may be administered, in the composition, alone or in combination with existing treatments. It may be administered for example alone and on the front line in the treatment of the disease, and associated with existing treatments in first or second line of treatment in the treatment of CLL. The goal is to prevent and counteract the therapeutic escape of patients. Advantageously, the antibody would be offered to refractory patients, for example CLL patients, at the first line of treatment who have suspended their therapy. Advantageously, immunotherapy is administered to elderly patients (> 70 years old) and/or to high-risk patients on the first line of treatment associated with existing therapeutic solutions.

Another object of the invention relates to a host cell that produces an isolated antibody of the invention.

Another object of the invention relates to an isolated nucleic acid sequence encoding an antibody of the invention, comprising a nucleic acid encoding at least one sequence of the group consisting of :
- a variable heavy (V_{H}) chain CDR 1 having the amino acid sequence SEQ ID NO: 3;
- a variable heavy (V_{H}) chain CDR2 having the amino acid sequence SEQ ID NO: 4;
- a variable heavy (V_{H}) chain CDR3 having the amino acid sequence SEQ ID NO: 5;
- a variable light (V_{L}) chain CDR1 having the amino acid sequence SEQ ID NO: 6;
- a variable light (V_{L}) chain CDR2 having the amino acid sequence SEQ ID NO: 7; and
- a variable light (V_{L}) chain CDR3 having the amino acid sequence SEQ ID NO: 8.

For example:
- nucleic acid sequence encoding a variable heavy (V_{H}) chain CDR 1 having the amino acid sequence SEQ ID NO: 3 may be SEQ ID NO: 11 (see figure 8),
- nucleic acid sequence encoding a variable heavy (V_{H}) chain CDR2 having the amino acid sequence SEQ ID NO: 4 may be SEQ ID NO: 12 (see figure 8);
- nucleic acid sequence encoding a variable heavy (V_{H}) chain CDR3 having the amino acid sequence SEQ ID NO: 5 may be SEQ ID NO: 13 (see figure 8);
- nucleic acid sequence encoding a variable light (V_{L}) chain CDR1 having the amino acid sequence SEQ ID NO: 6 may be SEQ ID NO: 14 (see figure 8);
- nucleic acid sequence encoding a variable light (V_{L}) chain CDR2 having the amino acid sequence SEQ ID NO: 7 may be SEQ ID NO: 15 (see figure 8);
- nucleic acid sequence encoding a variable light (V_{L}) chain CDR3 having the amino acid sequence SEQ ID NO: 8 may be SEQ ID NO: 16 (see figure 8);
- nucleic acid sequence encoding a variable light (V_{L}) chain having the amino acid sequence SEQ ID NO: 9 may be SEQ ID NO: 17 (see figure 8);
- nucleic acid sequence encoding a variable heavy (V_{H}) chain having the amino acid sequence SEQ ID NO: 10 may be SEQ ID NO: 18 (see figure 8).

Nucleic acid encoding the antibody, or a fusion protein comprising the antibody of the invention in the case of it is a fragment as described above, can be obtained by standard molecular biology or biochemistry techniques, such as DNA chemical synthesis, PCR amplification or cDNA cloning and can be inserted into expression vectors such that the genes are operatively linked to transcriptional and translational control sequences. In this context, the term "operatively linked" is intended to mean that a gene to express is ligated into a vector such that transcriptional and translational control sequences within the vector serve their intended function of regulating the transcription and translation of the gene. The expression vector and expression control sequences are chosen to be compatible with the expression host cell used. In case of an antibody or of a fusion protein, the genes encoding the different parts of the antibody can be inserted into separate vector or, alternatively, both genes are inserted into the same expression vector. The genes may be inserted into the expression vector by standard methods, such as ligation of complementary restriction sites on the gene fragment and vector.

Another object of the invention relates to an expression vector comprising a nucleic acid as defined above. The expression vector may be realized using the standard expression vectors available on the market. The expression vector comprise all the sequences necessary for the expression of the inserted genes. For example, in addition to the genes, the recombinant expression vectors of the invention may carry regulatory sequences that control the expression of the genes in a host cell. The term "regulatory sequence" is intended to include promoters, enhancers and other expression control elements, such as polyadenylation signals that control the transcription or translation of the antibody chain genes. The gene can be cloned into the vector such that the signal peptide is linked in frame to the amino terminus of the gene. The signal peptide can be an immunoglobulin signal peptide or a heterologous signal peptide, such as a signal peptide from a non-immunoglobulin protein. Regulatory sequences for mammalian host cell expression may include viral elements that direct high levels of protein expression in mammalian cells, such as promoters and/or enhancers derived from cytomegalovirus (CMV), Simian Virus 40 (SV40), adenovirus such as the adenovirus major late promoter (AdMLP), and polyoma. Alternatively, nonviral regulatory sequences may be used, such as the ubiquitin promoter or P-globin promoter. Still further, regulatory elements composed of sequences from different sources, such as the SRa promoter system, which contains sequences from the SV40 early promoter and the long terminal repeat of human T cell leukemia virus type 1. In addition to the antibody chain genes and regulatory sequences, the recombinant expression vectors of the invention may carry additional sequences, such as sequences that regulate replication of the vector in host cells, such as origins of replication, and selectable marker genes for facilitating selection of host cells into which the vector has been introduced. For example, typically the selectable marker gene confers resistance to drugs, such as G418, hygromycin or methotrexate, on a host cell into which the vector has been introduced. Selectable marker genes include the dihydrofolate reductase (DHFR) gene (for use in dhfr-host cells with methotrexate selection/amplification) and the neo gene (for G418 selection).

Another object of the invention relates to a method of producing an antibody of the invention, comprising culturing the host cell as defined above under conditions that result in production of the antibody of the invention, and isolating the antibody of the invention from the host cell or culture medium of the host cell.

The term "host cell" as used herein refers to a cell expressing the antibody of the invention, by transfection with a nucleic acid molecule or infection with phagemid or bacteriophage, and the progeny or potential progeny of such a cell. Progeny of such a cell may not be identical to the parent cell transfected with the nucleic acid molecule due to mutations or environmental influences that may occur in succeeding generations or integration of the nucleic acid molecule into the host cell genome. It may be any cell known in the prior art, for example SP2/0, YB2/0, IR983F, Namalwa human myeloma, PERC6, CHO-DG44, CHO-DUK-B11, CHO-K-1, CHO-Lec10, CHO-Lec1, CHO-Lec13, CHO Pro-5, CHO/DHFR-, Wil-2, Jurkat, Vero, Molt-4, COS-7, 293-HEK, BHK, K6H6, NS0, SP2/0-Ag 14 and P3X63Ag8.653.

For expression of the nucleic acid, the expression vector(s) may be transfected into the host cell by standard techniques. The various forms of the term "transfection" are intended to encompass a wide variety of techniques commonly used for the introduction of exogenous DNA into a prokaryotic or eukaryotic host cell, such as electroporation, calcium-phosphate precipitation, DEAE-dextran transfection and the like.

Cell culture production, purification and characterization of antibodies can be realized by well-known methods of the prior art. For example, cell can be allow to grow and die (4 to 5 days) before supernatant collection, clarification by low-speed centrifugation and volume reduction by ultrafiltration, for example on Pellicon XL Filter (Millipore). The concentrated culture supernatants can be injected into a HiTrap protein A FF column (GE Healthcare), bound antibodies can be eluted with sodium citrate buffer, and fractions can be neutralized using Tris. Fractions containing the antibodies can be pooled and dialyzed into PBS, and the samples can be sterile-filtered and stored at 4°C. The purified antibodies can be characterized by SDS-PAGE under non-reducing and reducing conditions.

Another object of the invention relates to a compound of the invention, for its use as a drug. In other words, it relates to the use of a compound of the invention as a drug.

Another object of the invention is a compound of the invention, for its use in treating a condition or a disorder in which the STIM1 protein localized to the plasma membrane of the cells is overexpressed. In other words, it relates to the use of a compound of the invention for the preparation of a drug for treating a condition or a disorder in which the amount of STIM1 protein localized to the plasma membrane of the cells is increased. For example, the condition or disorder may be selected from any pathology with an increase in mSTIM1 expression such as Systemic Lupus Erythematous or Chronic Lymphocytic Leukemia therefore any autoimmune disease, immunological, cancer, cardiovascular, muscular, neurological, hematological, inflammatatory, respiratory, infectious endocrine, cutaneous, gastrointestinal, metabolic, allergic diseases and transplantation with an increase in specific expression cells of mSTIM1

Another object of the invention relates to an isolated protein fragment consisting of the immunodominant STIM1 region containing the SAM domain between amino acid residues 97 and 127 of the STIM1 amino acid sequence SEQ ID NO: 1. The phrase "isolated protein fragment" as used herein refers to a protein fragment that has been identified and separated and/or recovered from a component of its natural environment, especially from the full sequence of the STIM1 protein.

Another object of the invention relates to the use of an isolated protein fragment of the invention, for immunization and producing an antibody that specifically binds to the region between amino acid residues 58 and 201, preferably between amino acid residues 97 and 127 of the STIM1 amino acid sequence SEQ ID NO: 1. Preferably the dominant epitopes of the invention is the peptide KLISVEDLWK.

Another object of the invention relates to the use of the protein fragment sequence of the invention between amino acid residues 58 and 201, preferably between amino acid residues 97 and 127 of the STIM1 amino acid sequence SEQ ID NO: 1 to develop STIM1 modulators including chemical components by virtual screening, inhibition assays, functional assays, binding assays or other techniques of the art.

This invention is further illustrated by the following examples with regard to the annexed drawings that should not be construed as limiting.

### Brief description of the figures

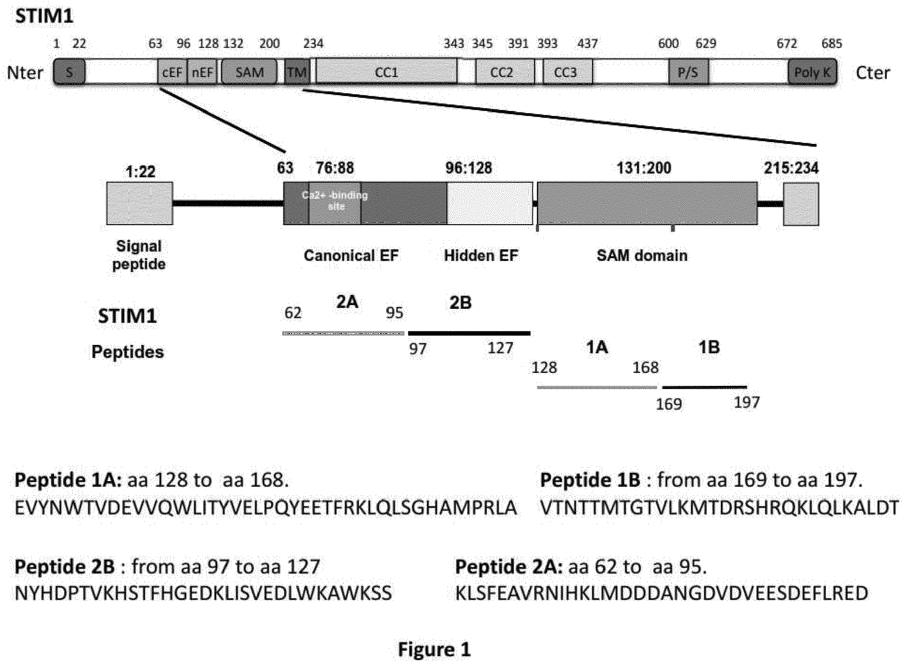

- Figure 1 presents the localization within the human STIM1 protein of the peptides designed for mice immunization realized to obtain the monoclonal anti-STIM1 antibody clone B-M4. Footpad immunization was realized for raising B-M4 mAb in mice. Four balb/c mice were immunized 5 times in footpad with 1 µg/mouse of mixed peptide 2A and peptide 2B. Three successive cloning steps were done to obtain a monoclonal hybridoma.

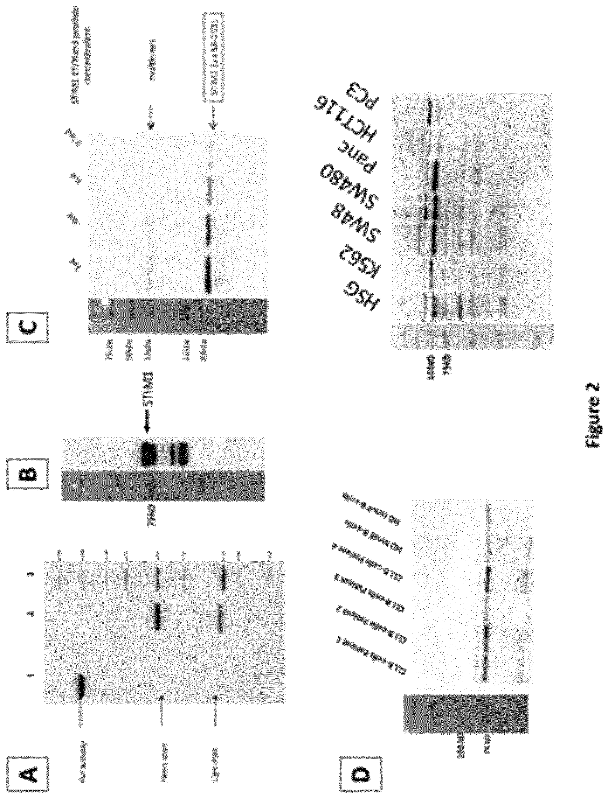

- Figure 2 illustrates the validation of monoclonal antibody anti-STIM1 clone B-M4 purity and specificity by Western Blot. Panel A: Lane 1: A single band at 150 kDa is observed in native condition corresponding to the full length B-M4 protein. Lane 2: Migration profile in denaturing conditions of anti-STIM1 antibody clone B-M4: band at 50 kDa and 25 kDa corresponds respectively to the heavy chain and to the light chain of the denaturated mAb in SDS page gel (4-15%) Lane 3 is the reference ladder. Panel B: Confirmation of STIM protein recognition by anti-STIM1 antibody B-M4 in PANC-1 cell line - Polyacrlamide gel (4-7,5%) were loaded with 75 µg protein deposit - anti-STIM1 antibody clone B-M4 mAb was diluted to 1/1000 (initial concentration: 2 mg/ml) and an anti-mouse IgG HRP (Abcam) diluted at 1/10 000 was used. Panel C: Confirmation of the STIM1 EF/Hand peptide (aa 58-201) recognition by the anti-STIM1 antibody clone B-M4. Polyacrylamide gel (4-7,5%) were loaded with 0,5 µg to 2 µg of the STIM1 EF/Hand peptide. Anti-STIM1 antibody clone B-M4 was diluted to 1/1000 (initial concentration: 2 mg/ml) and a anti-mouse IgG HRP (Abcam) 1/10 000) was use as a secondary antibody. Panel D: Confirmation of the STIM protein recognition by the anti-STIM1 antibody clone B-M4 in B cell lines, B cells from CLL patients, or B cells isolated from tonsils of healthy controls..Polyacrlamide gel (4-7,5%) was loaded with 75 µg of protein deposit - anti-STIM1 antibody clone B-M4 mab was diluted to 1/1000 (initial concentration: 2 mg/ml) and an anti-mouse IgG HRP (Abcam) 1/10 000 was used.

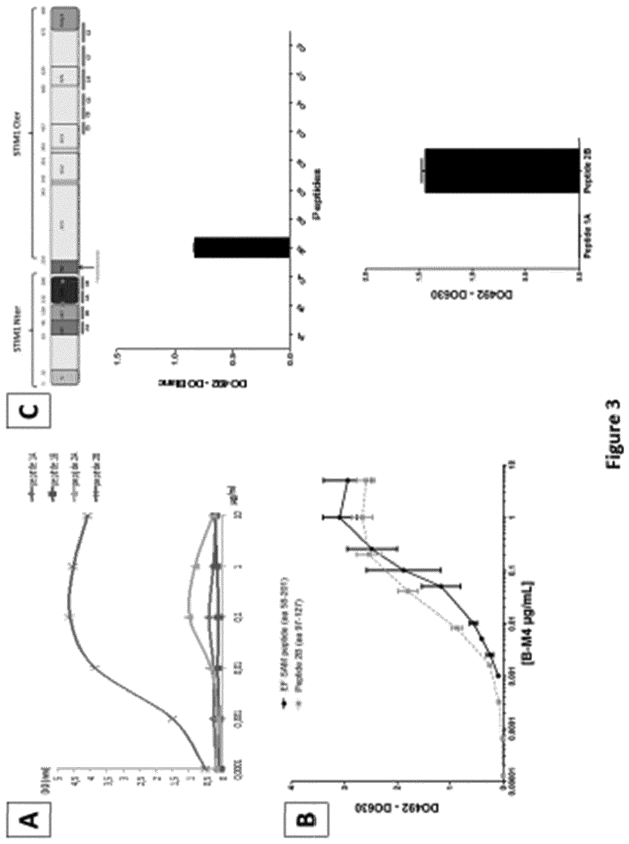

- Figure 3 illustrated the validation by Elisa of the monoclonal anti-STIM1 antibody clone B-M4. Panel A: Specificity of B-M4 antibody for the STIM1 peptide 2B is demonstrated by ELISA using the biotinylated STIM1 peptides used for immunization. Anti-STIM1 mAb clone B-M4 mAb (10 µg/ml) was diluted to 1/10 and antigens STIM1 biotinylated peptides used at concentrations of 50 ng/well for peptides 1A and 1B and 100 ng/well for peptide 2A and 10 ng/well for peptide 2B. Panel B: Specificity of the B-M4 antibody for STIM1 is confirmed by ELISA using the peptide 2B and a recombinant STIM1 EF/SAM peptide (1 µg/mL). B-M4 mAb used at 100 µg/mL diluted to 1/10. Panel C: Specificity for the B-M4 antibody for the peptide 2B over the other peptides (1A, 1B, 2A, 2B, C2, C3, C4, C5, C6, C7 and C8). Peptides are used at a concentration of 1 µg/mL. Histograms represents mean optical density measured using an Elisa approach.

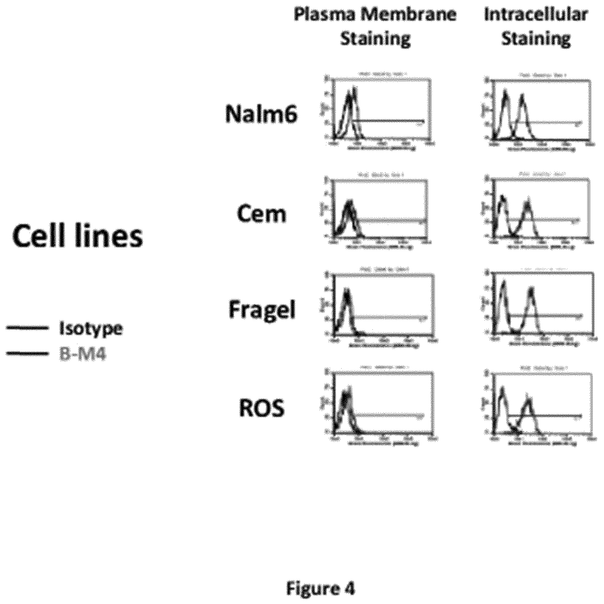

- Figure 4 shows the indirect detection of intra-cytoplasmic STIM1 and plasma membrane STIM1 (mSTIM1) in different cell lines by flow cytometry using the monoclonal Antibody anti-STIM1 clone B-M4 and a secondary antibody. B-M4 labeling was tested in parallel for membrane and intracytoplasmic staining on different cell lines (. An indirect staining was realized with a secondary antibody (GAM FITC). Labeling in cell lines show an intra-cytoplasmic labeling as well as a surface staining. In each condition, cells are incubated with 50 µl of purified antibody at 0.5 µg at +4°C during 30 min and then incubated with a secondary antibody (GAM FITC 30 min at +4°C) to reveal the staining.

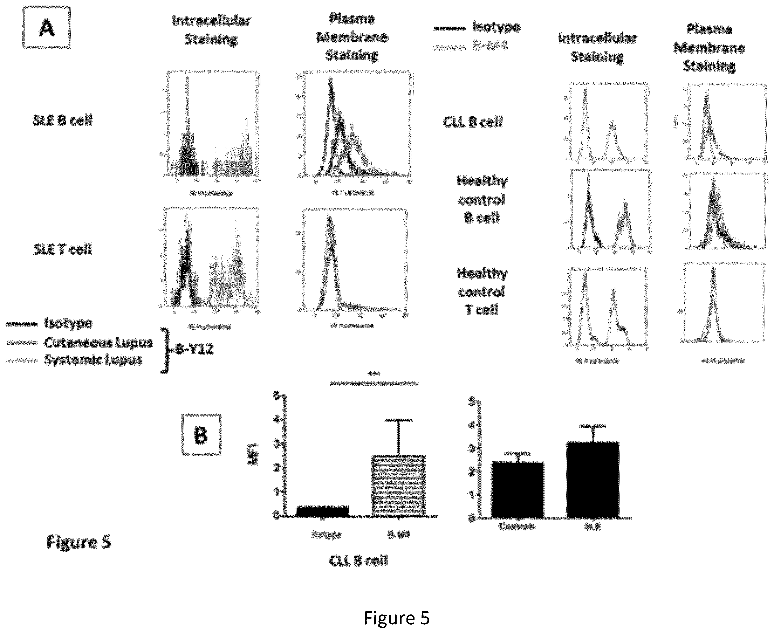

- Figure 5 represents the direct detection by flow cytometry of intra-cytoplasmic and plasma membrane fractions (mSTIM1) of STIM1 using the anti-STIM1 monoclonal antibody clone B-M4 coupled to Phycoerythrine (PE) in primary B cells from Systemic Erythematous Lupus (SLE) and Chronic Lymphocytic Leukemia (CLL) patients. Panel A: PE coupled B-M4 antibody was tested in parallel for membrane and intra-cytoplasmic staining in B and T cells from SLE or CLL patients and from healthy donors. Left side of panel A presents SLE and cutaneous lupus patients and CLL patients or healthy donors are presented on the right side of this panel A. 100 µl of whole blood cells (PBMC cells) were incubated with 100 µl of purified PE coupled antibody (2 µg/ml) at room temperature for 30 min during 30 min. Panel B presents the Mean Fluorescence Intensity (MFI) of mSTIM1 labeling obtained with the PE coupled B-M4 antibody in CLL (left side of panel B) and compares MFI from cells of healthy donor and LES patients (right side of panel B). Data are analyzed by non-parametric Wilcoxon matched-pairs analysis, ***p<0.001 for control and LES patient. Data are analyzed by non-parametric Mann Withney analysis, ***p<0.001 for LLC.

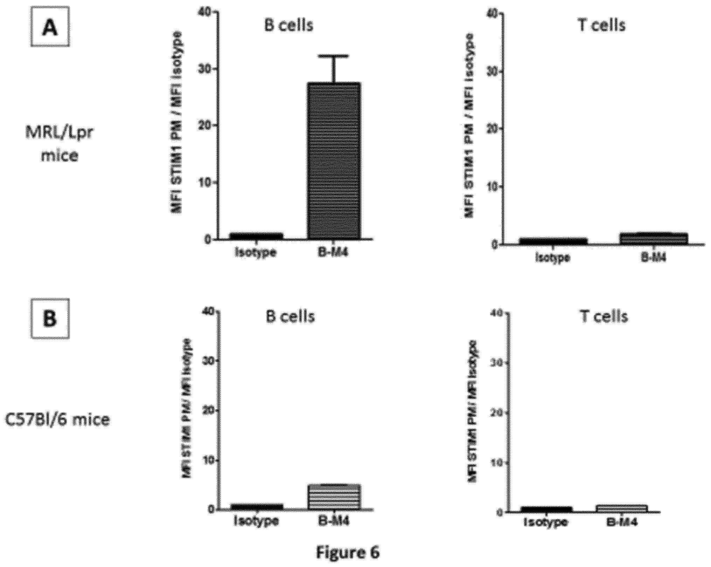

- Figure 6 represents the validation by flow-cytometry of the anti-STIM1 antibody clone B-M4 specificity for the STIM1 protein. Plasma membrane STIM1 (mSTIM1) is detected in B and T cells isolated from spleen of mice direct detection by flow cytometry using Monoclonal Antibody anti-STIM1 clone B-M4 coupled to Phycoerythrine (PE). Panel A: Labelling of mSTIM1 by PE coupled B-M4 mAb in B and T cells from MRL/Lpr mice (n=4). Mean Fluorescence Intensity (MFI) of mSTIM1 labeling with PE coupled B-M4 is compared to labeling with an isotype control. Panel B: Labeling of mSTIM1 by PE coupled B-M4 mAb in B and T cells from C57BI/6 mice (n=3). Mean Fluorescence Intensity (MFI) of mSTIM1 labeling with PE coupled B-M4 is compared to labling obtained with an isotype control.

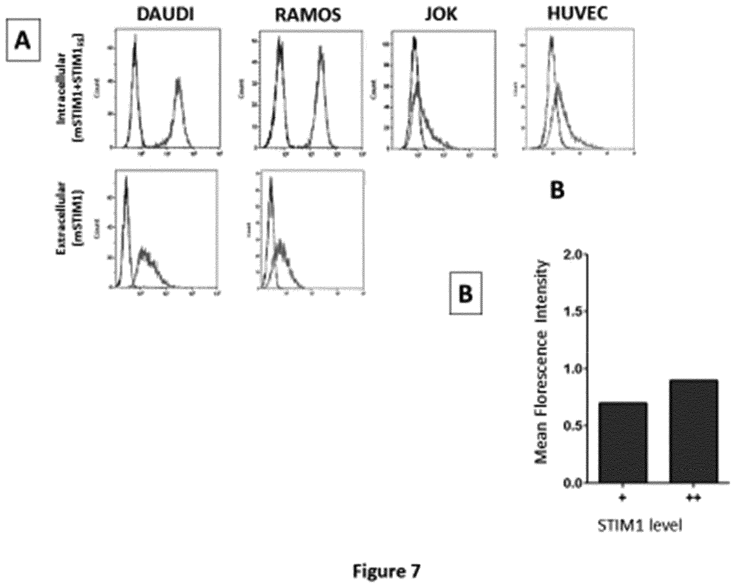

- Figure 7 represents the direct detection by flow cytometry of the intra-cytoplasmic and plasma membrane (mSTIM1) fractions of STIM1 in cells line using the anti-STIM1 monoclonal antibody (mAb) clone B-M4 coupled to Phycoerythrine (PE). Panel A: Labeling of STIM1 using the PE coupled B-M4 mAb in DAUDI, RAMOS and JOK B cell lines and in endothelial cell line HUVEC. Cells were either left intact to labeled mSTIM1 or permeablized to label total STIM1 (mSTIM1 + intracellular STIM1). 100µl of cells were incubated with the PE coupled B-M4 antibody (2 µg and 4 µg for membrane and intracellular staining respectively), at 4°C during 30 min. Panel B presents the Mean Fluorescence Intensity (MFI) of mSTIM1 labeling with the PE coupled B-M4 antibody in HEK293 cells over-expressing STIM1 (++) compared to cells transfected with an empty vector (+). Cells are incubated with PE coupled B-M4 antibody (2 µg) 4°C during 30 min.
- Figure 8 represents the sequences corresponding to complete variables light and heavy chains of the anti-STIM1 antibody clone B-M4. Total RNA is extracted and reverse transcription of the RNA (5'CDS primer) was done. Amplification of variable chains by RACE-PCR (various reverse primer) and cloning of the amplicons in shuttle vector were done. The figure shows the sequences after analysis of the complementarity determining region (CDR) in heavy chain variable domain (nucleotide and amino acid sequence) and light chain variable domain (nucleotide and amino acid sequence)..

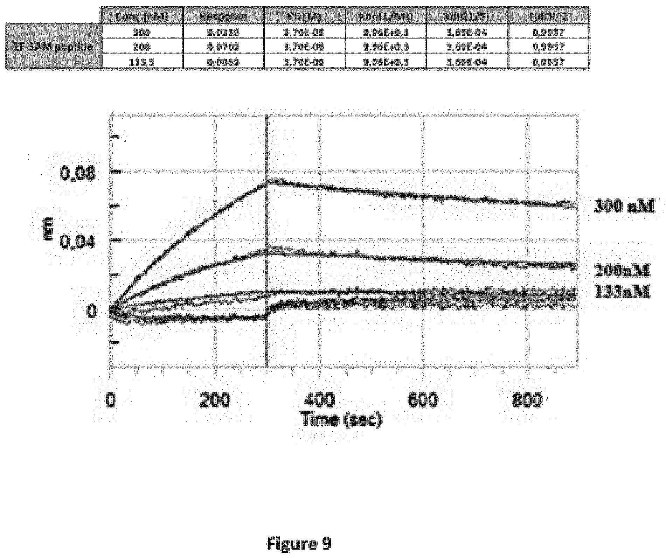

- Figure 9 represents the determination of the anti-STIM1 mAb clone B-M4 affinity. Evaluation of the B-M4 mAb affinity for the soluble recombinant protein corresponding to the STIM1 EF-Hand domain (amino-acids 59 to 201) was determined using the Octet technology. The KD (kdis/kon) was determined using a 1 to 1 fitting model. EF-SAM peptide (STIM1 aa 58 to 201) was tested at 300, 200 and 133 nM. High affinity interaction is characterized by a low K, a fast recognizing (high kon) and a stable formation of complexes (low kdis). kdis: dissociation rate constant. kon: complexe formation rate.

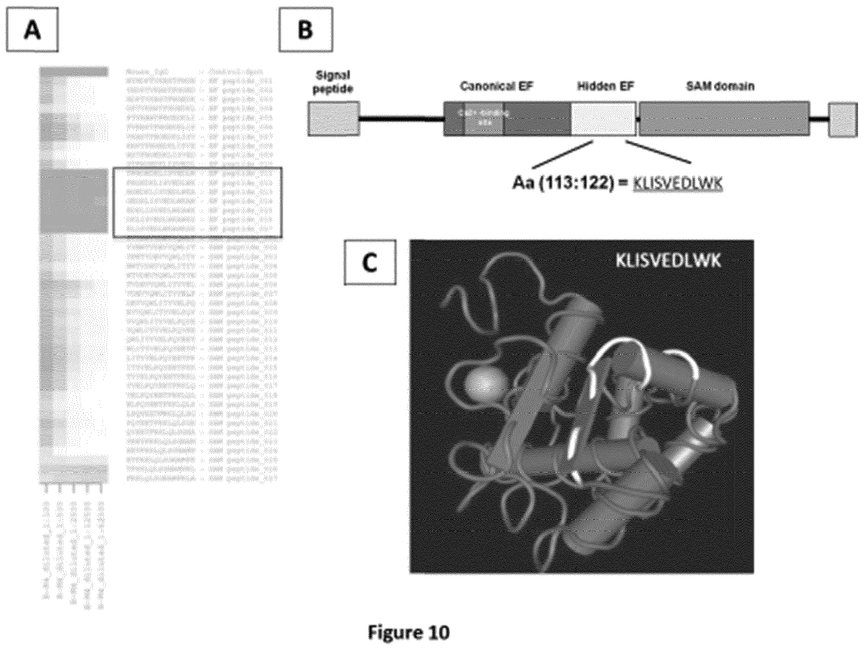

- Figure 10 represents the epitope Identification for the anti-STIM1 antibody clone B-M4. Epitope mapping was realized by epitope detection using overlapping peptide scans (Pepscan). The heatmap diagram after scanning of all the overlapping peptides is presented in Panel A. Representation shows the fluorescence intensities as direct measurement of antibody binding in a grey-coded manner from white (no binding) to black (strong binding). The anti-STIM1 aantibody clone B-M4 demonstrates highly significant interactions with the common sequence "KLISVEDLWK" that represents the epitope of this antibody. The localisation of this sequence within the N terminus STIM1 protein sequence is represented in panel B. The projection of this sequence (white line) is displayed in the 3D structure of the N terminal extracellular part of STIM1 protein (Panel C).

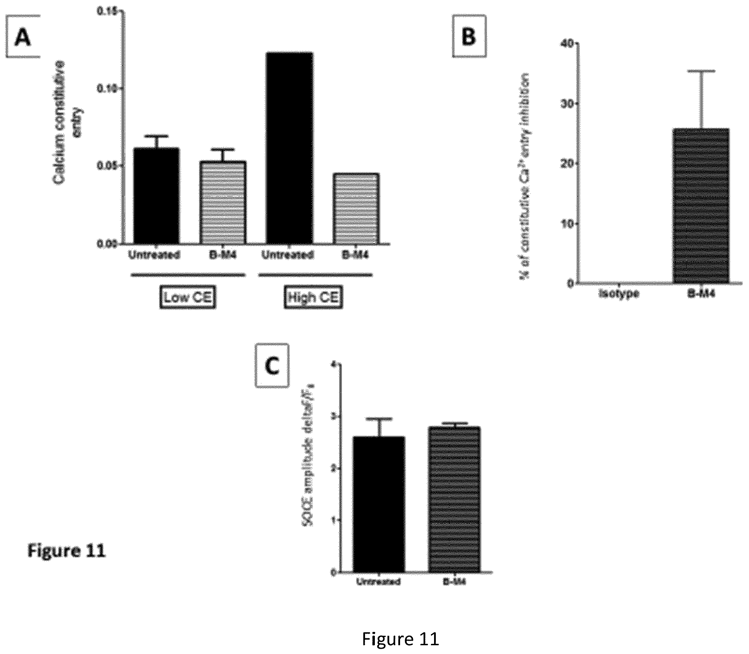

- Figure 11 represents the inhibition of Constitutive Ca²⁺ entry (CCE) by anti-STIM1 mAb clone B-M4 in B cells. Panel A illustrates the Inhibition by the B-M4 anti-STIM1 mAb of Constitutive Ca²⁺ entry from purified B lymphocytes isolated from CLL patients with either low or high CCE. Cells are incubated for 1H with 10 µg/ml of the B-M4 mAb. CCE was revealed by changing external Ca²⁺ concentration from 5 mM to 0,5 mM and the amplitude of CCE evaluated by the difference in normalized fluorescence ratio when changing external Ca²⁺ concentration. Average amplitudes of constitutive entry (CCE) are presented. Panel B: Inhibition of CCE from HEK cell line by the B-M4 anti-STIM1 mAb. Cells are incubated for 1H with 10 µg/ of the B-M4 mAb. The percentage (%) of CCE inhibition compared to the isotype treatment is presented. Panel C: Store Operated Ca²⁺ entry measured in a B cell line (JOK cells) cells is not modulated by the anti-STIM1 mAb clone B-M4. SOCE is recorded after endoplasmic reticulum Ca²⁺ store depletion with Thapsigargin (2 µM) in 0 mM external Ca²⁺ and re-addition of 1.8 mM extracellular Ca²⁺. JOK cells were incubated with an isotype or with the anti-STIM1 antibody. Histograms display the individual values of SOCE amplitude mean values +/- SEM.

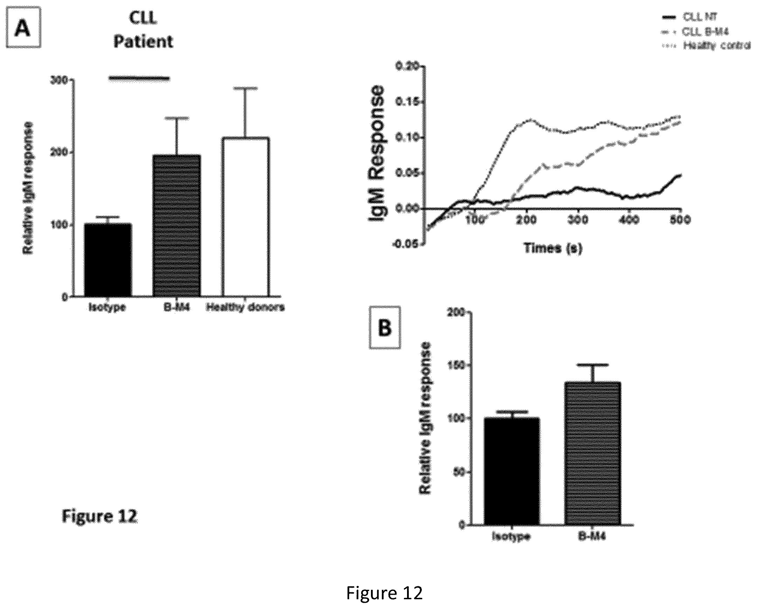

- Figure 12 represents the modulation signals by anti-STIM1 mAb clone B-M4 of the BCR induced Ca²⁺ signal. Panel A illustrates that the anti-IgM induced Ca²⁺ response are in part corrected by the B-M4 antibody in B-cells from CLL patients with reduced BCR induced Ca²⁺ responses but not in B cells from healthy donors (left side of panel A). Amplitude of Ca²⁺ responses is measured after anti-IgM stimulation of B-cells incubated or not for 1h with B-M4 antibody. The Amplitude of the Ca²⁺ transient is compared to what measured in B cells from healthy donors. Panel B displays the amplitude of Ca²⁺ responses measured after Anti-IgM stimulation of Jok cell line incubated for 1h with B-M4 mAb or its isotype. IgM stimulation is realized with 10 µM of polyclonal goat anti-human IgM.

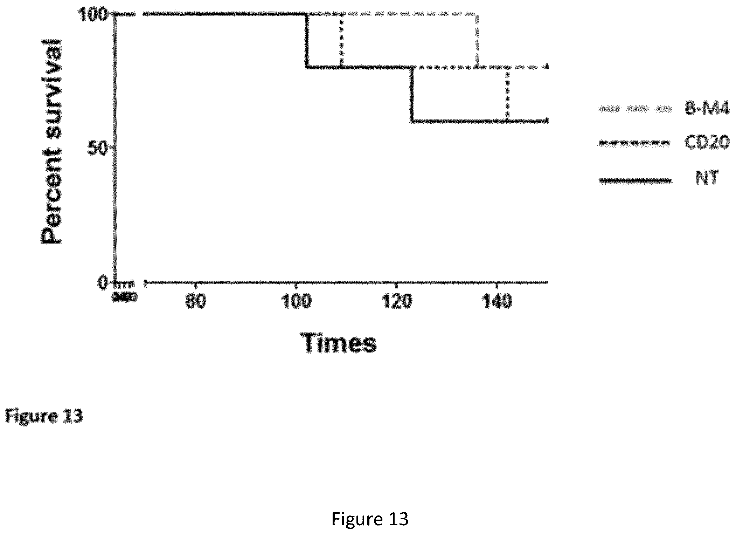

- Figure 13 represents the demonstration of the beneficial effect of anti-STIM1 mAb clone B-M4 treatment on the survival of lupus prone mice MRL/Lpr. Survival is greatly increased in mice injected twice a week with the anti-STIM1 mAb B-M4 (5 mg/Kg; n=15 mice) compared to mice injected with mAb anti-CD20 (2.5 mg/Kg; n=15 mice) and mice injected with PBS buffer. Data are analyzed by non-parametric Log-rank (Mantel-Cox) Wilcoxon matched-pairs analysis, * p<0.05.

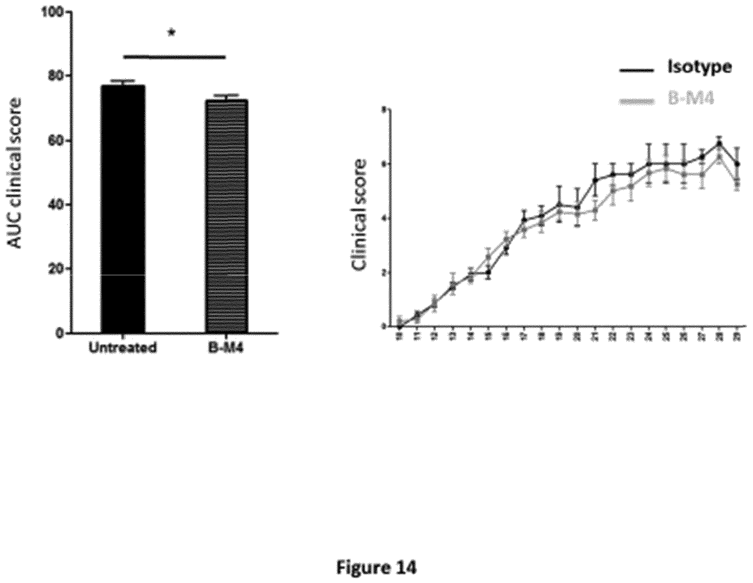

- Figure 14 represents the beneficial effect on a clinical score of lupus prone mice (MRL/Lpr) treatment with the anti-STIM1 mAb clone B-M4. The clinical score is defined by addition of the lymph node hypertrophic score (normal = 0, moderate = 1, severe = 2), the cutaneous score (alopecia = 1, ulceration =2) and the score of mice pain (moderate = 2 and severe = 4). The clinical score is significantly reduced in mice injected twice a week with the anti-STIM1 mAb clone B-M4 (10 µg) compared to mice injected with mAb isotype (IgG1, 10 µg). Histograms represents the average of the area under the curve (AUC) of the clinical score evolution over time. Data are analyzed by Welch's unpaired t analysis *p<0.05

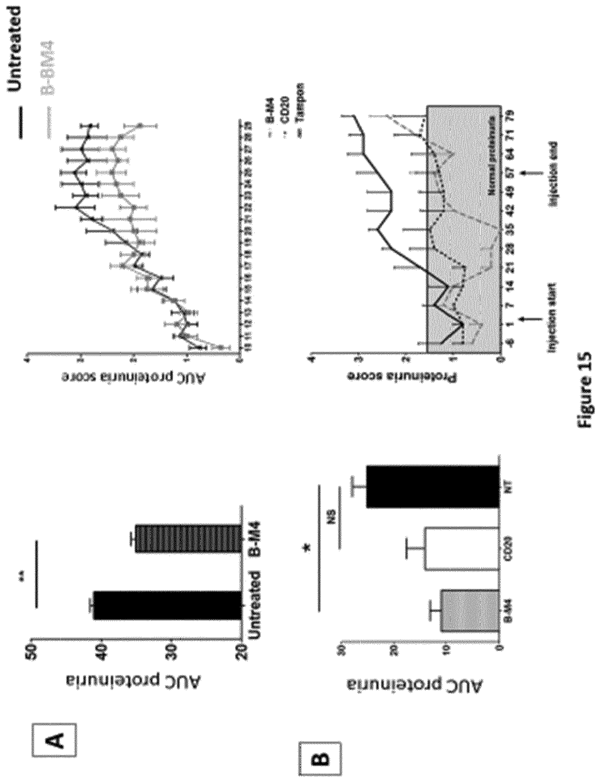

- Figure 15 displays the demonstration of the beneficial effect on the proteinuria of lupus prone mice MRL/Lpr mice of the anti-STIM1 mAb clone B-M4 treatment. Panel A shows that the proteinuria score is reduced in mice injected twice a week with the anti-STIM1 mAb B-M4 (0.3 mg/kg) compared to mice injected with the PBS buffer. Histogram represents the average of the area under the curve (AUC) of proteinuria evolution over time. Panel B illustrates that proteinuria score is significantly reduced in mice injected twice a week with anti-STIM1 mAb clone B-M4 (5 mg/kg) compared to mice injected with anti-CD20 mAb (2.5 mg/kg) and to non-treated mice. Histogram represents the average of the area under the curve (AUC) for proteinuria evolution over time. Urine samples were tested for proteinuria using Multistix 10 SG on a 0-4+ scale, corresponding to the following approximate protein concentrations: 0, negative or trace; 1+, 30 mg/dl; 2+, 100 mg/dl; 3+, 300 mg/dl; and 4+, 2000 mg/dl. Mice were considered to have severe nephritis if two consecutive urine samples scored 3+. Data are analyzed by Welch's unpaired t analysis ***p<0.001; *p<0.05*

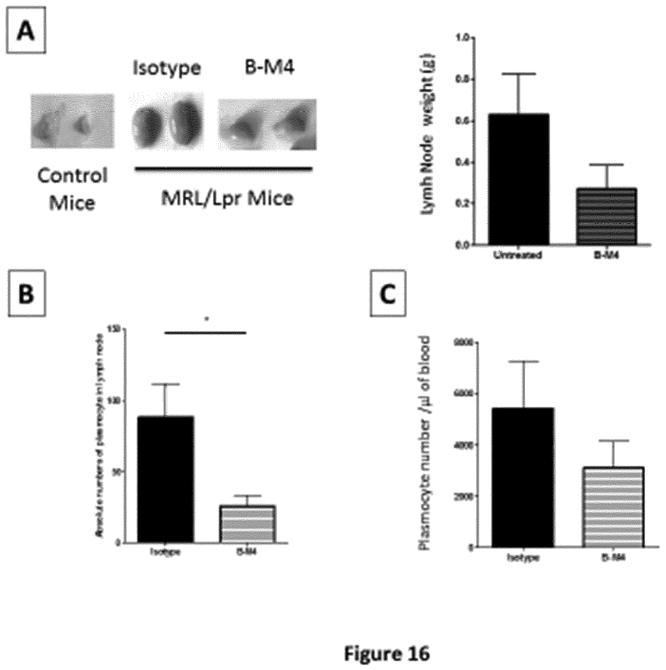

- Figure 16 represents the beneficial effects of the treatment of lupus prone mice MRL/Lpr mice with the monoclonal antibody anti-STIM1 clone B-M4 on lymphoproliferation. Panel A shows that injection of MRL/Lpr mice with anti-STIM1 mAb B-M4 (10 µg) twice a week reduces lymph node size and weight compared to the injection of mice with mAb isotype (IgG1, 10 µg). The number of plasma cells in lymph node (Panel B) and in the blood (Panel C) is reduced. Lymph node cells and blood leukocytes were incubated with 5 µg/ml of rat anti-mouse CD16/CD32 and incubated with the appropriate Abs CD138. Data are analyzed by non-parametric Mann Whitney analysis, *p<0.05.

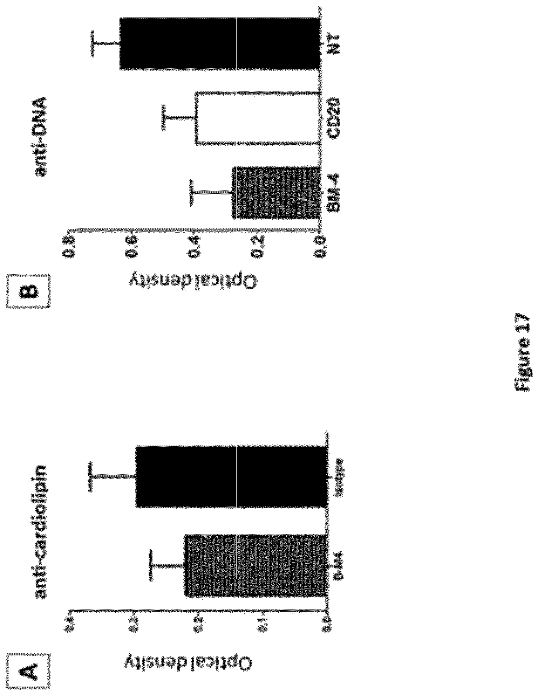

- Figure 17 illustrates the beneficial effects of monoclonal antibody anti-STIM1 clone B-M4 treatment on the auto-immune symptoms of lupus prone mice MRL/Lpr. Anti-STIM1 clone B-M4 treatment reduces auto-antibody production in lupus injected mice. Panel A illustrates that injection twice a week of MRL/Lpr mice with the anti-STIM1 mAb clone B-M4 (10 µg) reduces the amount of anti-phospholipid (cardiolipin) auto-antibodies detected in the blood of injected mice. Autoantibodies were detected by Elisa with Cardiolipin in serum diluted to 1/100. Panel B shows that Injection of MRL/Lpr mice with anti-STIM1 mAb B-M4 (5 mg/Kg) or anti-CD20mAb (2.5 mg/Kg) twice a week reduces the amount of anti-DNA autoantibodies were detected by Elisa with salmon sperm and in serum diluted to 1/1000.

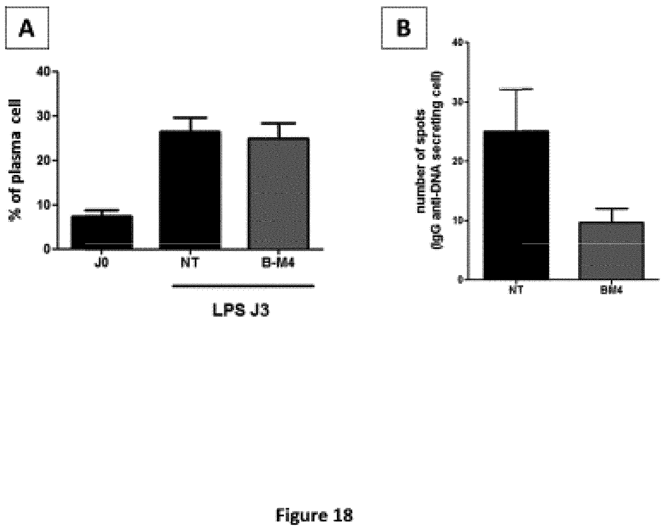

- Figure 18 illustrates the *in vitro* inhibition effect of the monoclonal antibody anti-STIM1 clone B-M4 on B-cell differentiation to autoreactive plasma cells. MRL/Lpr mice B cell differentiation into plasma cell was performed by stimulating B cells for 4 days with LPS (10 µg/mL) Cells were treated or not for these 4 days with the anti-STIM1 mAb clone B-M4 (10 µg/mL). Cells were stained with specific antibodies to evaluate plasma cell number (CD138+) by flow cytometry **(Panel A)** and the number of cells secreting DNA autoantibodies cell were evaluated by Elispot **(Panel B**

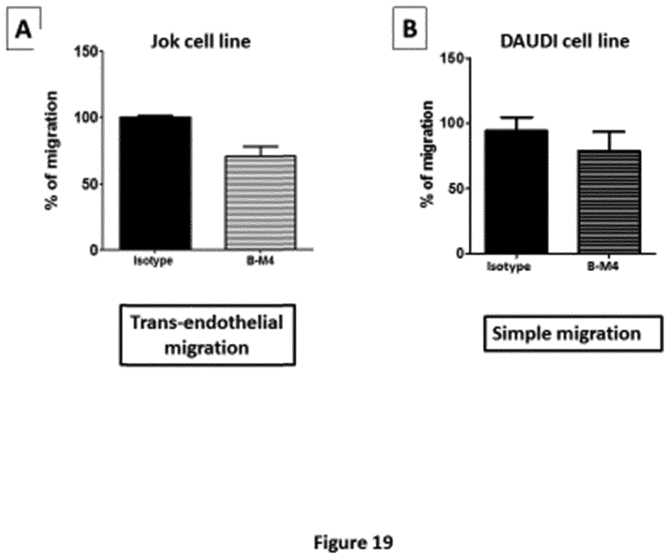

- Figure 19 illustrates that the *In vitro* migration of B cells is inhibited by the anti-STIM1 mAb clone B-M4. B cell migration experiments were realized with Boyden Chambers and migration was stimulated by a CCL12 chemokine gradient. Anti-STIM1 clone B-M4 mAb (10 µg/ml) inhibits both the trans-endothelial migration through an endothelial cell layer (HUVEC cells) of JOK B cell line (Panel A) and the migration through a filter of DAUDI cell line (Panel B).

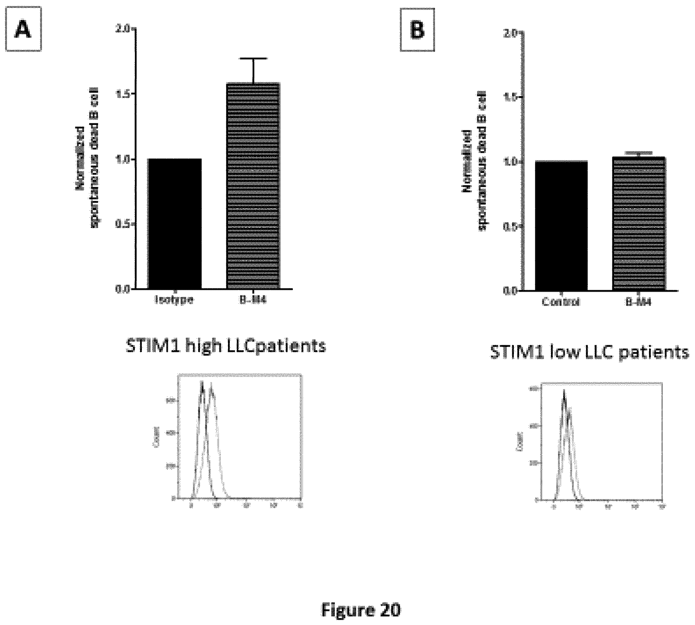

- Figure 20 shows that the anti-STIM1 mAb clone B-M4 affects B cell survival *in vitro.* Treatment with B-M4 mAb (100 µg/mL) of isolated B cells from CLL patients displaying a high level of plasma membrane STIM1 (mSTIM1) reduces B cells survival after 48h (Panel A) whereas B cells with low amount of mSTIM1 are not affected (**Panel B**). Membrane staining with PE coupled B-M4 was performed to evaluate mSTIM1 expression in B cells isolated from CLL patients. Cell survival (Annexin V-/PI-) was evaluated by Anexin V/PI staining in flow cytometry

### Examples

### Example 1: Process of preparation of the monoclonal antibody B-M4

An antibody of the invention (named "B-M4") was obtained by immunization of mice injected with two peptides corresponding to the SAM domain of the STIM1 protein: peptides 2B (SEQ ID NO: 2) and 2A (SEQ ID NO: 19, KLSFEAVRNIHKLMDDDANGDVDVEESDEFLRED corresponding to amino acids 169 to 197 of STIM1) (see Figure 1). Footpad simultaneous immunization with both peptides was realized for raising anti-STIM1 antibody clone B-M4 mAb in mice. Four balb/c mice were immunized 5 times in footpads with 1 µg/mouse of mixed peptide 2A and peptide 2B. Three successive cloning steps were done to obtain a monoclonal hybridoma.

By Western blot and ELISA approaches, it has been demonstrated that the antibody B-M4 is a specific antibody recognizing STIM1 and more specifically the region of this protein corresponding to amino acids (aa) 97 to 127 of the SAM domain of STIM1 protein (see Figure 2 and Figure 3).

Protein extraction was performed on 10⁷ B cells for 30 on ice with a lysis buffer containing: 20 mM Tris HCl pH 7.5, 150 mM NaCl, 1 mM EDTA, 1 mM EGTA, 1% Triton X100, 2.5 mM Na+ pyrosodium tetraphosphate, 1 mM glycerophosphate, 1 mM Na+ orthovanadate, 1 µg/ml leupeptin and a protease inhibitor cocktail. Protein extracts were sonicated and centrifuged for 12 min at 16,000 g. Protein concentration of cell lysates were determined using the Folin method. 75 µg of proteins were run on SDS-PAGE 7,5 % polyacrylamide gels in denaturing conditions, and then transferred onto PVDF (PolyVinyliDene Fluoride) membrane sheets. Unspecific blocking was done by incubation with 5% fat milk in PBS, 0,1 % tween 20 for 1 hour. Blots were incubated overnight with 5% fat milk in PBS, 0.1% tween 20, containing mouse monoclonal anti-STIM1 (clone: BY-12, 1:1,000 dilution) or mouse monoclonal anti-GAPDH antibody (6C5 clone Abcam; 1:10,000 dilution,). Blots were incubated with Horseradish Peroxydase (HRP)-conjugated goat anti-mouse after washing with PBS, 0,1 % tween 20 and revealed with the Luminata Forte reagent. All results were normalized upon GAPDH quantification.

For the ELISA measurements, 5x 10⁶ cells were loaded for 1 hour on 96 wells pre-coated CellTak plates. Cells were fixed using PFA 4% for 10 min at room temperature (RT). Cells were then washed with Phosphate Buffer Solution (PBS) and next incubated with PBS supplemented with 5% of fat milk for 30 minutes. Cells were next incubated with the anti-STIM1 antibody directed against the N terminus (clone: B-Y12, 1 µg/ml) for 1h30 at RT. After 3 washes with PBS, cells were incubated with in PBS +5% of fat milk containing the peroxidase conjugated secondary antibody for 30 min at RT. After 3 washes, the substrate for peroxidase conjugated secondary antibody (SIGMA*FAST*™ OPD tablets, Sigma-Aldrich) was added for 20 min at 37°C and the reaction was stopped using H₂SO₄ solution. ELISA plate was read at 392 nm in absorbance.

By Flow cytometry, direct or indirect recognition by monoclonal antibody B-M4 of the plasma membrane-bound STIM1 protein (mSTM1) or the endoplasmic reticulum membrane has been demonstrated in different cell lines (see Figure 4) as well as in healthy donor B lymphocytes or patients with SLE or CLL (see Figure 5) and mice B cells (see figure 6). The specificity of mSTIM1 labeling by clone B-M4 was confirmed in flow cytometry by labeling STIM1 in cells expressing different levels of this protein (see Figure 7). This antibody makes it possible to specifically detect, in flow cytometry, the membrane fraction of STIM1 (mSTIM1) as well as the majority fraction of STIM1 located at the reticulum membrane.

5 x 10⁶ B cells were used per condition. Cells were either left intact or permeablized to labeled mSTIM1 or total STIM1. B cells were centrifuged for 5 min at 1500 rpm and incubated with 100 µL of PBS containing anti-STIM1 antibody directed against the N terminus (clone: PE coupled B-Y12; 2 µg for mSTIM1 and 4 µg for total STIM1 or 0,5 µL of an isotype control for 30 min on ice. After 3 washes, cells were read in PBS using a Flow cytometer (Navios, Beckman Coulter Life Sciences).

The variable domains of the heavy and light chains of the B-M4 antibody have been sequenced (see Figure 8). The isotype of clone B-M4 is IgG1 / Kappa.

Total RNA is extracted and reverse transcription of the RNA (5'CDS primer) was done. Amplification of variable chains by RACE-PCR (various reverse primer) and cloning of the amplicons in shuttle vector were done. Sequences of the complementarity determining region (CDR) in heavy chain variable domain (nucleotide and amino acid sequence) and light chain variable domain (nucleotide and amino acid sequence) were analysis.

The affinity of this B-M4 mAb for the STIM1 protein was determined by the octet technology using a peptide corresponding to the EF-SAM domain of this protein (aa: 58 to 201). In the conditions used, B-BM4 has a KD of 3.7.10⁻⁸ (see Figure 9).

Evaluation of the anti-STIM1 mAb affinity for the soluble recombinant protein corresponding to the STIM1 EF-Hand domain (amino-acids 59 to 201) was determined using the Octet technology (Octet™). The KD (kdis/kon) was determined using a 1 to 1 fitting model. EF-SAM peptide (STIM1 aa 58 to 201) was tested at at 300, 200 and 133 nM. Affinity constants (Kon, Kdis) were calculated.

The epitope of the antibody anti-STIM1 clone B-M4 was identified as linear epitope with the sequence "KLISVEDLWK ». Epitope mapping was realized by epitope detection using overlapping peptide scans (Pepscan).

### Example 2: Effect of the monoclonal antibody B-M4 on the constitutive entry of Ca²⁺ of B lymphocytes from patients suffering from CLL or LES

The antibody B-M4 inhibits the constitutive entry of Ca²⁺ of B lymphocytes from patients suffering from CLL or SLE. This blockage of constitutive entry is also observed in cell lines (HEK293, B-Cell Lines) (see Figure 11).

For constitutive Ca²⁺ entry (CCE) measurements, 5x 10⁶ B cells were loaded with 2 µM of the Fura-2/AM fluorescent dye in the presence of 2 µM Pluronic acid for 30 min at 37°C in a medium containing: 135 mM NaCl, 5 mM KCI, 1 mM MgCl₂, 10 mM HEPES, 10 mM Glucose with an 7,4-adjusted pH supplemented with 5 mM CaCl₂. Cells were washed and left to attach in the same buffer on 12mm CellTaK precoated coverslides for 20 min. Fura-2 was excited alternatively at 340 and 380 nm using a monochromator, and fluorescence emission was recorded at 510 nm using a fluorescence microscope equipped with a dichroic mirror and a 14-bit CCD camera. After the stabilization of basal fluorescence, the extracellular medium was replaced by Buffer A supplemented with 0.5 mM CaCl₂ for 100 s and again with the original 5mM CaCl₂-containing Buffer A after curve stabilization. Values of the ratio of fluorescence measured at 340 and 380 nm are collected over time and normalized.

This antibody has no effect on activated Ca²⁺ entry by the effect of thapsigargin on the release of intracellular calcium stores (SOCE: Store Operated Ca²⁺ Entry) (see Figure 11). Anti-STIM1 mAb clone B-M4 does not modulate BCR induced Ca²⁺ signals but increase this signal in CLL cells with high level of mSTIM1 (see figure 12).

For SOCE measurement 5x 10⁵ cells were seeded in precoated CellTak 96 wells. Cells are loaded with Fura-2 acetoxymethyl ester (Fura-2 QBT™, Molecular Probes) fluorochrome according to the manufacturer's protocol. The Fura-2 QBT™ was aspirated and replaced by an equal volume of free Ca²⁺ Hepes-buffered solution containing (in mM): 135 NaCl, 5 KCI, 1 MgCl₂, 1 EGTA, 10 Hepes, 10 glucose, pH adjusted at 7.45 with NaOH. Intracellular calcium level variations were monitored by using the FlexStation 3™ (Molecular Devices, Berkshire, UK), Dual excitation wavelength capability permits ratiometric measurements of Fura-2AM peak emissions (510 nm) after excitations at 340 nm (bound to Ca²⁺) and 380 nm (unbound to Ca²⁺). Modifications in the 340/380 ratio reflect changes in intracellular-free Ca²⁺ concentrations. The SOCE was elicited by releasing the Ca²⁺ stores from the endoplasmic reticulum with thapsigargin (2 µM) solution under Ca²⁺-free conditions to determine the magnitude of intracellular Ca²⁺ release (Hepes-buffered solution). Next, cells were returned to a Ca²⁺-containing Hepes-buffered solution to measure SOCE. The magnitude and speed of SOCE were estimated. Igm stimulation to stimulate BCR induced Ca²⁺ signals is realized with 10 µM of a polyclonal goat anti-human IgM in the presence of in 2 mM external Ca²⁺ and in a solution containing (in mM): 135 NaCl, 5 KCI, 1 MgCl₂, 1 EGTA, 10 Hepes, 10 glucose, pH adjusted at 7.45 with NaOH.

### Example 3: Biological effect of the monoclonal antibody B-M4 on MRL / Lpr mice

The anti-STIM1 clone B-M4 antibody treatment increases MRL/Lpr lupus prone mice survival compared to mice injected with buffer or a reference treatment such as anti-CD20 antibody (see Figure 13). Anti-STIM1 clone B-M4 antibody treatment decreases the clinical score indicative of the general condition of the mice injected with this antibody compared to the mice injected with an isotype (see Figure 14).

Only MRL/Lpr female mice were used in this work. Mrl/Lpr lupus prone mice were injected twice a week with anti-STIM1 mAb B-M4 (5 mg/Kg) compared to mice injected with mAb anti-CD20 (2.5 mg/Kg) and mice injected with PBS buffer. Clinical score is defined by addition of lymph node hypertrophy score (normal = 0, moderate = 1, severe = 2) and cutaneous score (alopecia = 1, ulceration =2) and the score of pain of mice (moderate = 2 and severe = 4). Mrl/Lpr lupus prone mice were injected twice a week with anti-STIM1 mAb B-M4 (10 µg) compared to mice injecteor injected with PBS buffer.

Anti-STIM1 B-M4 antibody decreases renal damage in MRL / Lpr mice. These disorders result from the exacerbated production of autoantibodies in these mice (autoimmune symptoms). The treatment of the MRL / Lpr mice with the anti-STIM clone B-M4 antibody induces in particular a reduction in the increase of the proteinuria in these mice compared to the mice injected with a control isotype (see FIG. 15).

MRL/Lpr mice were injected with anti-STIM1 mAb B-M4 (10 µg) with or PBS buffer twice a week. Urine samples were tested for proteinuria using Multistix 10 SG (Bayer Diagnostics, Puteaux, France) on a 0-4+ scale, corresponding to the following approximate protein concentrations: 0, negative or trace; 1+, 30 mg/dl; 2+, 100 mg/dl; 3+, 300 mg/dl; and 4+, 2000 mg/dl.

The treatment of lupus mice with the B-M4 antibody decreases the lymphoproliferation observed in these MRL / Lpr mice (see Figure 14). Injection of the B-M4 antibody reduced the size and weight of the lymph nodes of these mice compared to mice injected with a control isotype. The injection with the B-M4 antibody greatly reduces the number of ganglion infiltrating plasmocytes as well as the number of plasma cells present in the blood (see Figure 16). Anti-STIM1 B-M4 antibody reduces the production of autoantibodies (anti-cardiolipin) in mice MRL / Lpr (see Figure 17).

MRL/Lpr mice were injected with anti-STIM1 mAb B-M4 (10 µg) with or the mAb isotype (lgG1, 10 µg) or PBS buffer twice a week. Lymph node size was measured and their weight was evaluated for each mice. The number of plasma cells in lymph node and in the blood was evaluated. Lymph node cells and blood leukocytes were incubated with 5 µg/ml of rat anti-mouse CD16/CD32 and incubated with the appropriate Abs CD138 to identify and count plasma cells by flow cytometry. Autoantibodies were detected in mice sera diluted to 1/100 by Elisa with Cardiolipin coated on place. Autoantibodies against DNA were detected using mice serum diluted to 1/1000 by Elisa with salmon sperm coated on plates.

### Example 5: Effect of the monoclonal antibody B-M4 on differentiation of B-lymphocytes

Anti-STIM1 B-M4 antibody inhibits the in vitro differentiation of MRL/Lpr B-lymphocytes (see Figure 18). The anti-STIM1 B-M4 antibody inhibits autoantibodies secretion (see Figure 18).

B cells were positively sorted from murine splenocytes by using a CD19 isolation kit. B cells were cultured at a concentration of 1 X 10⁶ / mL in RPMI containing 10% FCS, L-glutamine, penicillin/streptomycin, B cells were stimulated with LPS (lipopolysaccharides) and incubated or not with 10 µg/mL of B-M4 antibody. After 3 days in culture, 2 X 10⁵ cells were cultured in Elispot plate and cells were staining with the appropriate CD138 antibody to count the number plasma cells by flow cytometry The number of anti DNA IgG secreting cells was evaluated using the classical Elispot protocol.

Anti-STIM1 B-M4 antibody reduces in vitro migration of B-cells (see Figure 19).

B cell migration experiments were realized with Boyden Chambers and migration was stimulated by a CCL12 chemokine gradient. Trans-endothelial migration of JOK B cell line was evaluated by measuring the migration of these cells through an endothelial cell monolayer of HUVEC cells cultured on a 5 µM pore filter. Simple migration of B cells was evaluated by measuring the migration of DAUDI B cells and B cells from CLL patients through a 5 µM pore filter.

B cells were treated all along the 24 hours of migration time with 10 µg/ml of the anti-STIM1 clone B-M4 mAb. The number of migrating cells was evaluating by counting the number of B cells in the lower chamber of the boyden chamber after 24 hours using flow cytometry. For each experimental condition, the percentage of migrated cells is normalized to what measured in the untreated control condition.

Anti-STIM1 B-M4 antibody reduces in vitro survival of B lymphocytes (see Figure 20).

B cells were incubated with 10 µg/ml of the anti-STIM1 clone B-M4 mAb for 48h and cell survival was evaluated at the end of 48 hours by Anexin/PI staining in flow cytometry. For mSTIM1 detection and quantification in B cells isolated from CLL patients, membrane staining of B cells was realized with PE coupled B-M4 mAb in flow cytometry

### Reference List

1. EP2982982.
2. EP3062105.
3. Morrison et al., Proc. Natl. Acad. Sci. U.S.A., 81, pp. 6851-55 (1984).
4. Harlow et al.: "Antibodies: A Laboratory Manual", Cold Spring Harbor Laboratory Press, 2nd ed. 1988.
5. Hammerling, et al.: "Monoclonal Antibodies and T-Cell Hybridomas", Elsevier, N. Y., 1981, pp. 563-681.
6. Jones et al, Nature, 321: 522-525 (1986).
7. Riechmann et al, Nature, 332: 323-329 (1988).
8. Presta, Curr. Op. Struct. Biol. 2: 593-596 (1992).

## Claims

1. A compound that specifically binds to the region between amino acid residues 58 and 201 of the STIM1 amino acid sequence SEQ ID NO: 1 and modulates STIM1 activity.

2. A compound according to claim 1 that specifically binds to the region between amino acid residues 97 and 127 of the STIM1 amino acid sequence SEQ ID NO: 1 and modulates STIM1 activity.

3. A compound according to claim 1, said compound being an activator or an inhibitor of STIM1 activity.

4. A compound according to claim 1, said compound being chosen from the group comprising isolated antibodies or fragments thereof, proteins, peptides, chemical or natural compounds, aptamers or any biological compound.

5. A compound according to claim 3, said compound being an isolated antibody.

6. An isolated antibody according to claim 6, comprising at least one sequence of the group consisting of:
- a variable heavy (V_{H}) chain complementary determining region (CDR) 1 having the amino acid sequence SEQ ID NO: 3;
- a variable heavy (V_{H}) chain CDR2 having the amino acid sequence SEQ ID NO: 4; and
- a variable heavy (V_{H}) chain CDR3 having the amino acid sequence SEQ ID NO: 5.

7. An isolated antibody according to claim 5 or 6, comprising at least one sequence of the group consisting of:
- a variable light (V_{L}) chain CDR1 having the amino acid sequence SEQ ID NO: 6;
- a variable light (V_{L}) chain CDR2 having the amino acid sequence SEQ ID NO: 7; and
- a variable light (V_{L}) chain CDR3 having the amino acid sequence SEQ ID NO: 8.

8. An isolated antibody according to anyone of claims 5 to 7, comprising:
- a variable heavy (V_{H}) chain CDR 1 having the amino acid sequence SEQ ID NO: 3;
- a variable heavy (V_{H}) chain CDR2 having the amino acid sequence SEQ ID NO: 4;
- a variable heavy (V_{H}) chain CDR3 having the amino acid sequence SEQ ID NO: 5;
- a variable light (V_{L}) chain CDR1 having the amino acid sequence SEQ ID NO: 6;
- a variable light (V_{L}) chain CDR2 having the amino acid sequence SEQ ID NO: 7; and
- a variable light (V_{L}) chain CDR3 having the amino acid sequence SEQ ID NO: 8.

9. An isolated antibody according to anyone of claims 5 to 8, comprising :
a) a variable light chain comprising the sequence SEQ ID NO: 9; and
b) a variable heavy chain comprising the sequence SEQ ID NO: 10.

10. An isolated antibody according to anyone of claims 5 to 9, which is a monoclonal antibody.

11. An isolated antibody according to anyone of claims 5 to 10, which is a chimeric, human or humanized antibody.

12. An isolated antibody according to anyone of claims 5 to 11, which is modified to be coupled with a drug, for example for enhancing ADCC, or with another antibody, or with a fluorophore, or with any other molecules or ligand such as nanoparticles, metals or radioelements, for imaging, and/or for therapeutic and/or for theranostic use.

13. A composition comprising a therapeutically effective amount of a compound according to anyone of the preceding claims.

14. A host cell that produces an isolated antibody according to anyone of claims 5 to 12.

15. An isolated nucleic acid sequence encoding an antibody as defined in anyone of claims 5 to 12, comprising a nucleic acid encoding at least one sequence of the group consisting of :
- a variable heavy (V_{H}) chain CDR 1 having the amino acid sequence SEQ ID NO: 3;
- a variable heavy (V_{H}) chain CDR2 having the amino acid sequence SEQ ID NO: 4;
- a variable heavy (V_{H}) chain CDR3 having the amino acid sequence SEQ ID NO: 5;
- a variable light (V_{L}) chain CDR1 having the amino acid sequence SEQ ID NO: 6;
- a variable light (V_{L}) chain CDR2 having the amino acid sequence SEQ ID NO: 7; and
- a variable light (V_{L}) chain CDR3 having the amino acid sequence SEQ ID NO: 8.

16. An expression vector comprising a nucleic acid as defined in claim 15.

17. A method of producing an antibody as defined in anyone of claims 5 to 12, comprising culturing the host cell as defined in claim 14 under conditions that result in production of said antibody, and isolating said antibody from the host cell or culture medium of the host cell.

18. A compound as defined in anyone of claims 1 to 12, for its use as a drug.

19. A compound as defined in anyone of claims 5 to 12, for its use in treating a condition or a disorder in which the STIM1 protein localized to the plasma membrane of the cells is overexpressed.

20. A compound for its use according to claim 18 or 19, wherein the condition or disorder is selected from a pathology with an increase in specific cells of mSTIM1 expression such as Systemic Lupus Erythematous or Chronic Lymphocytic Leukemia therefore any autoimmune disease, immunological, cancer, cardiovascular, muscular, neurological, hematological, inflammatatory, respiratory, infectious endocrine, cutaneous, gastrointestinal, metabolic, allergic diseases and transplantation with an increase in specific expression cells of mSTIM1.

21. An isolated protein fragment consisting of the region between amino acid residues 97 and 127 of the STIM1 amino acid sequence SEQ ID NO: 1.

22. Use according to claim 21 wherein said compound specifically binds to the region between amino acid residues 97 and 127 of the STIM1 amino acid sequence SEQ ID NO: 1.

23. Use of the protein fragment as defined in claim 22, for developing compounds that specifically binds to the region between amino 97 and 127 of the STIM1 amino acid sequence SEQ ID NO: 1 and modulates STIM1 activity.
